# EUROPEAN PATENT SPECIFICATION

(11) **EP 1 644 484 B1**
(45) Date of publication and mention of the grant of the patent: **17.09.2008**
(21) Application number: 04701001.2
(22) Date of filing: 09.01.2004
(51) Int. Cl.: C12N 5/06, C12N 5/08

(54) **AUTOLOGOUS SELF-TOLERANCE INDUCING CELLS OF MONOCYTIC ORIGIN AND THEIR USE IN PHARMACEUTICAL PROPARATIONS**
AUTOLOGOUS SELBST-TOLERANZ INDUZIERENDE ZELLEN MONOZYTÄREN URSPRUNGS UND IHREN VERWENDUNG IN ARTZNEIMITTELN
CELLULES AUTOLOGUES D'ORIGINE MONOCYTIQUE INDUISANT UNE AUTOTOLERANCE ET LEUR UTILISATION DANS DES PREPARATIONS PHARMACEUTIQUES

(30) Priority: 11.07.2003 WO PCT/EP03/07551
(43) Date of publication of application: 12.04.2006
(73) Proprietor: Blasticon Biotechnologische Forschung GmbH, 24063 Kiel (DE)
(72) Inventor: KREMER, Bernd, Karl, Friedrich, 24107 Kiel (DE); FÄNDRICH, Fred, 24105 Kiel (DE); SCHULZE, Maren, 24238 Wittenberger Passau (DE)
(74) Representative: UEXKÜLL & STOLBERG
(86) International application number: PCT/EP2004/000109
(87) International publication number: WO 2005/005620

(56) References cited:
- WO-A-02/097070
- WO-A-03/083092
- WO-A-20/04007701
- RONCAROLO M-G ET AL: "The role of different subsets of T regulatory cells in controlling autoimmunity" CURRENT OPINION IN IMMUNOLOGY, CURRENT BIOLOGY LTD, XX, vol. 12, no. 6, 1 December 2000 (2000-12-01), pages 676-683, XP004257742 ISSN: 0952-7915
- MUNN DAVID H ET AL: "Selective activation-induced apoptosis of peripheral T cells imposed by macrophages: A potential mechanism of antigen-specific peripheral lymphocyte deletion" JOURNAL OF IMMUNOLOGY, vol. 156, no. 2, 1996, pages 523-532, XP002287396 ISSN: 0022-1767
- LOPEZ M ET AL: "Infusion of large quantities of autologous blood monocyte-derived macrophages in two cancer patients did not induce increased concentration of IL-6, TNF-alpha, soluble CD14 and nitrate in blood plasma" EUROPEAN CYTOKINE NETWORK, vol. 5, no. 4, 1994, pages 411-414, XP008032591 ISSN: 1148-5493
- RUTELLA SERGIO ET AL: "Role for granulocyte colony-stimulating factor in the generation of human T regulatory type 1 cells" BLOOD, vol. 100, no. 7, 1 October 2002 (2002-10-01), pages 2562-2571, XP002287397 ISSN: 0006-4971

## Description

The invention relates to autologous cells of monocytic origin, which are capable of inducing immunologic self-tolerance in a patient. These cells are subsequently designated "STIC" (self-tolerance inducing cells). The invention further relates to the use of STIC in pharmaceutical preparations for the prevention and/or treatment of diseases associated with disturbed self-tolerance, as in particular autoimmune diseases and allergies.

In relation to the invention the term "autologous" indicates that the STIC are derived from monocytes from the blood of the respective patient to whom the STIC are to be administered.

It has been shown by the inventors that the cells of the invention are capable of inducing regulatory T-cells (Treg_{CD4+25+}). The invention therefore also relates to the induction and/or *in vitro* preparation of regulatory T-cells.

The immune system protects the body from potentially pathogenic antigens, as for instance microorganisms, while it normally avoids reacting with constituents of the body itself; i.e. the healthy immune system tolerates "self antigens".

Disturbed self-tolerance occurs when specific adaptive (acquired) immune responses are mounted against self antigens.

The normal consequence of an adaptive immune response against the foreign antigen is the clearance of the antigen from the body. When an adaptive immune response develops against self antigens, it is, however, usually impossible for immune effector mechanisms to eliminate the antigen completely and so a sustained response occurs. The consequence is that the effector pathways of immunity cause chronic inflammatory injury to tissues, which may prove lethal (see Immuno Biology 5, The Immune System In Health and Disease, Garland Publishing 2001, Chapter 13, pages 501-522).

Adaptive immune responses are initiated by the activation of antigen-specific T and/or B cells, and it is believed that autoimmunity is initiated the same way (Immuno Biology, loc.cit. p. 501).

One preferred embodiment of the invention relates to the treatment and/or prevention of autoimmune diseases using pharmaceutical preparations containing STIC.

Two major patterns of autoimmune diseases may be distinguished. Diseases, in which the manifestation of autoimmunity is restricted to specific organs of the body, are known as "organ-specific" autoimmune diseases, while, in "systemic" autoimmune diseases, many tissues of the body are affected. Examples of organ-specific autoimmune diseases are Hashimoto's thyroiditis and Graves' disease, which each predominantly affect the thyroid gland, and Type I insulin-dependent diabetes mellitus, which affects the pancreatic islets. Examples of systemic autoimmune diseases are systemic lupus erythematosus and primary Sjögren's syndrome, in which tissues as diverse as the skin, kidneys, and brain may all be affected (see Immuno Biology, loc.cit, page 503).

There are autoimmune diseases, which are primarily believed to be T cell-mediated, as for instance insulin-dependent diabetes mellitus, rheumatoid arthritis, and multiple sclerosis, while, in other cases, the formation of antibodies to cell surface or matrix antigens plays a dominant role, as for example in autoimmune hemolytic anaemia, autoimmune thrombocytopenic purpura, Goodpasture's syndrome, pemphigus vulgaris or acute rheumatic fever; a still other group are immune-complex diseases, wherein both T cells and B cells are involved, as for instance mixed essential cyroglobulinemia, systemic lupus erythematosus or rheumatoid arthritis (see Immuno Biology, loc.cit, Fig. 13.1 on page 502).

A further embodiment of the invention relates to the treatment of allergies with the STIC of the invention formulated as pharmaceutical preparations.

It has recently been suggested that regulatory T-cells play an important role in controlling immune homeostasis, that is, an orchestrated immune response to any infectious or antigen related target including immunologic self-tolerance, see Takeshi Takahashi and Shimon Sakaguchi, International Review of Cytology, 225, 1-32 (2003); Shimon Sakaguchi, Vox Sang 83, 151-153 (2002), Kathryn J. Wood and Shimon Sakaguchi, Nature Reviews Immunology, 3. 199-210 (2003).

As stated by Takahashi et al., loc.cit. page 1, Abstract, "Accumulating evidence indicated that T cell-mediated dominant control of self-reactive T cells contributes to the maintenance of immunologic self-tolerance and its alternation may lead to development of autoimmune disease. Efforts to delineate such regulatory T cell populations have revealed that CD 25⁺ cells within the CD4⁺ population in normal naive animals including humans possess the regulatory activity. The CD25⁺ plus CD4⁺ regulatory T cells are produced by the normal thymus as a functionally distinct subpopulation of T cells. They play critical roles not only in preventing autoimmunity but also in controlling various immune reactions".

In addition to T cell mediated autoimmune diseases, the B cell compartment of the immune system can trigger autoaggressive diseases associated with the production of antibodies directed against self cells (including mast cells), tissue and organ structures.

It is well known that B cell activation depends on T cell help in such a way that T helper cells stimulate clonal B cell expansion upon presentation of specific antigens. Those antigens may be derived from fragmented allergens and are then processed within the T cell to small molecule peptides. These are then presented in a MHC-restricted fashion in order to stimulate antigen-specific activation.

In order to prevent uncontrolled or excessive B cell activation caused by specific allergens which leads to allergic diseases on the one hand and which can induce disturbed self-tolerance on the other hand (see above), regulatory T cells have the capacity to interfere with T cell associated B cell activation and prevent excessive and uncontrolled antibody production.

Similar to autoimmune diseases, also allergic diseases can therefore be influenced and controlled by increasing the amount of regulatory T cells (CD4⁺/CD25⁺ T cells). In particular, it has been shown in mice suffering from EAE ("experimental allergic encephalomyelitis") that the disease remits a couple of weeks after CD4⁺ T cells have been administered to these animals, and that the cure is associated with resistance to any further disease induction (Bach, J.F. "Regulatory T Cells under Scrutiny" Nature Reviews Immunology 3: 189-198 (2003); Lando, Z. et al. "Effect of cyclophosphamide on suppressor-cell activity in mice unresponsive to EAE" J. Immunol. 123: 21556-2160 (1979)). These effects are similar to those shown for NOD mice in which the progression of autoimmune diabetes can be stopped by administration of CD4⁺ cells, further leading to protection of the mice against new outbreaks of the autoimmune disease (Bach, J.F., *loc.cit*.).

Examples for allergic diseases, which may be associated with autoimmune reactions are all types of allergies induced by non-self proteins, organic and inorganic substances encountering the body. Of particular importance in this regard are allergies induced by pollen like e.g. hayfever and allergies induced by allergens like drugs, chemicals, viruses, bacteria, fungi, house dust, food components, metals, gas, body components of animals such as skin scale or hair, and animal excreta.

To date no effective therapeutics are available for the prevention and/or treatment of diseases resulting from disturbed self-tolerance.

With respect to organ specific autoimmune diseases, substitution treatment, transplantation or symptomatic treatment with antiinflammatory agents like cortisone are usually applied. With respect to systemic autoimmune diseases, immuno suppressive agents are often used for treatment. Clearly these "therapies" are problematic in many respects and suffer from serious side effects.

It is estimated that up to 5% of the population are afflicted by autoimmune diseases (Sakagushi, loc.cit., page 151, left column). Therefore, an urgent need exists for effective means for the prevention and/or treatment of diseases associated with disturbed self-tolerance which are easy to handle and which are not associated with the health threatening side effects and the high costs involved with the methods and agents to date applied in the treatment of such diseases.

It is therefore the problem underlying the invention to provide improved means for preventing and/or treating diseases associated with disturbed self-tolerance.

For the solution of the problem, the use of autologous self-tolerance inducing cells (STIC) of monocytic origin from vertebrates, in particular from mammals and more preferred from humans is proposed. These cells are obtainable by the process of the invention described below, which process results in modified cells, capable of increasing the amount of regulatory T-lymphocytes (CD4⁺/CD25⁺ T-cells) in the body of an individual. After administration of approximately 10⁵ cells/kg body weight(BW), these cells are.suitable for preventing and/or treating diseases associated with disturbed self-tolerance.

M. Lopez et al., European Cytokine Network, vol. 5, no. 4, 1994, pages 411-414 report the use of autologous blood monocytes to increase the number of autologous macrophages for reinfusion in cancer immunotherapy trials. Reinfusion of high numbers of autologous macrophages was well tolerated, and no increase in TNF-alpha, IL-6 or soluble CD14 levels was observed in patient's plasma (page 413, left column, 1^{st} and 2^{nd} para.).

### Description of the Figures

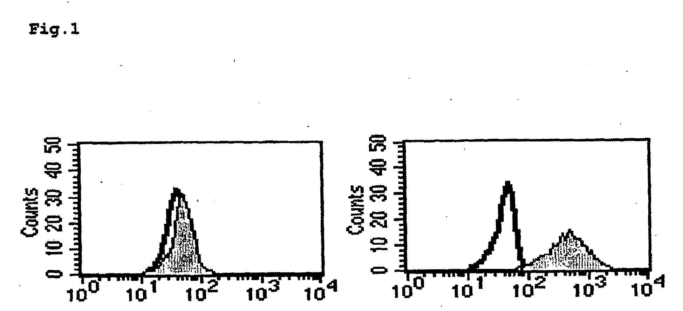

- Figure 1:: Flow cytometric determination of the binding capacity of GM-7 to original monocytic cells before (graph on the left hand side) and after (graph on the right hand side) modification of the cells according to the invention. The x axis indicates the number of bound cells.

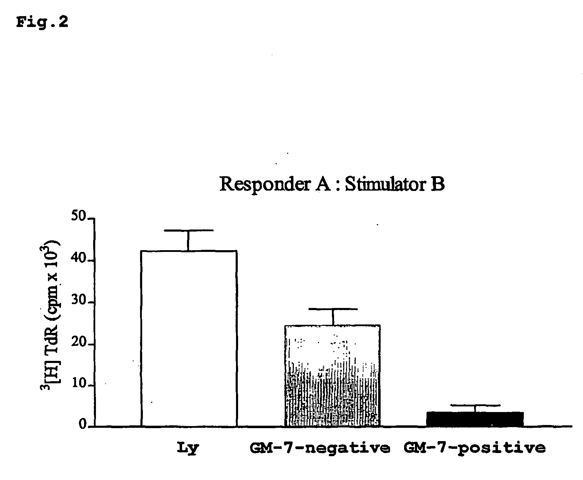

- Figure 2:: Mixed lymphocyte culture of CD14⁺ monocytes from an individual B (GM-7⁻: grey column; GM-7⁺: black column), responder cells from an MHC-discordant donor A and irradiated cells from a donor B to compare the suppressor activity of CD14⁺/GM-7⁺ and CD14⁺/GM-7⁻ cells.

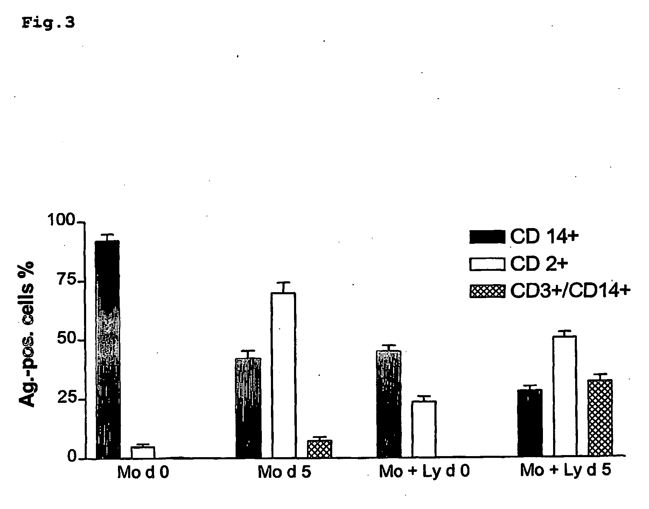

- Figure 3:: Flow cytometric determination of the amount of CD14⁺-monocytes and of CD2⁺-lymphocytes in the monocyte fraction as well as the amount of the CD-14⁺/CD3⁺-cells effective as TAIC, for determining the influence of purifying the cells for enriching monocytes at the beginning of the culture on the formation of immunosuppressive CD14⁺/CD3⁺-cells.

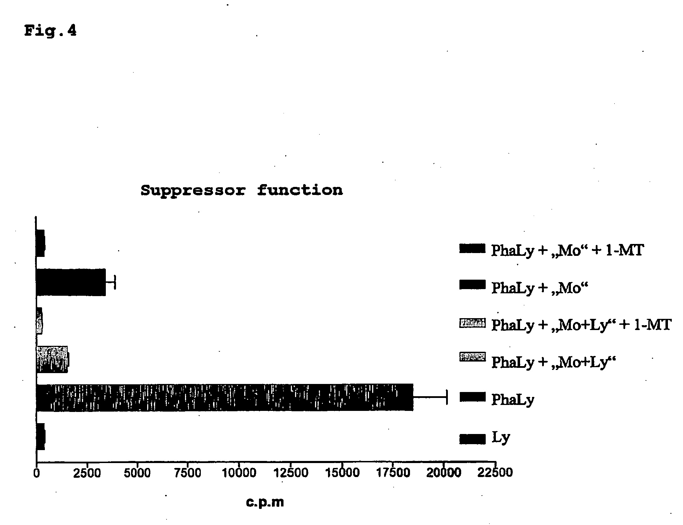

- Figure 4:: Mixed lymphocyte culture of PHA-stimulated lymphocytes (PhaLy) and TAIC ("Mo+Ly" or "Mo"), preincubated in two experiments with an inhibitor (1-MT) of indolamine-2,3-dioxygenase (IDO) for determining the influence of 1-MT on the suppressor activity of the TAIC.

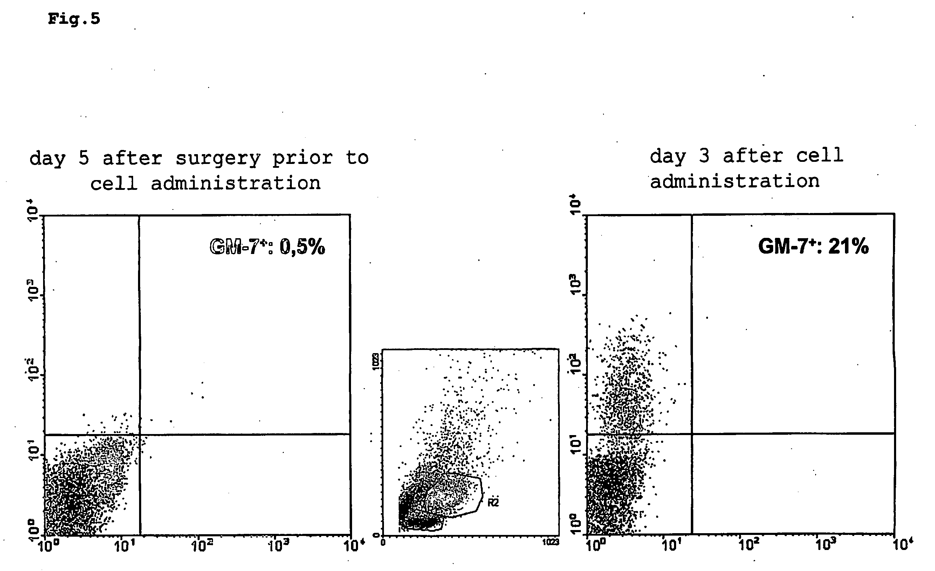

- Figure 5:: Flow cytometric determination of GM-7-expression in the blood of patients postoperatively prior (left Figure) and after (right Figure) injection of TAIC, for determining the influence of TAIC on the in vivo-GM-7-expression in blood cells.

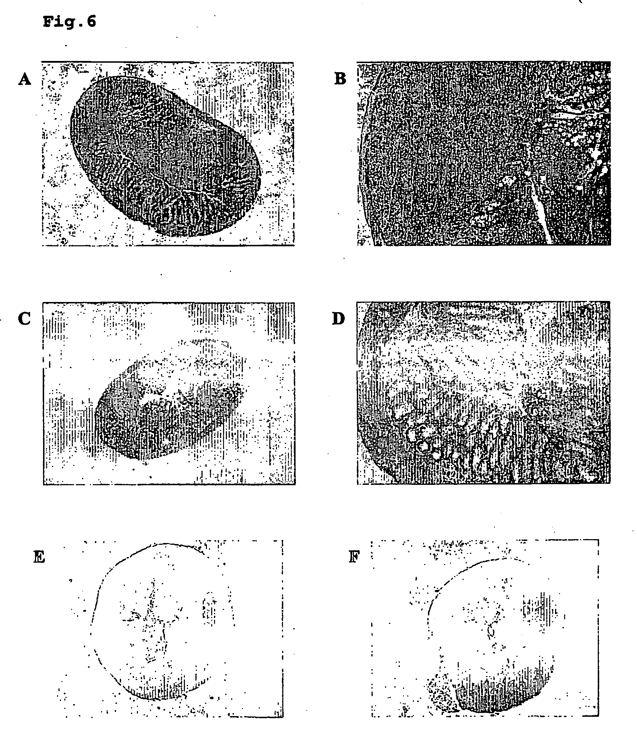

- Figure 6:: H&E staining of colon sections of mice suffering from dextran sulphate sodium (DSS)-induced chronic colitis illustrating the condition of the colon of an untreated animal of group 3 (Fig. 6A/B), an animal of group 1 treated with STIC on day +1 following the DSS treatment (Fig. 6C/D), an animal of group 4 treated with "control cells" at day +1 (Fig. 6E) and of an animal treated with STIC at day +7 (Fig. 6F). (magnification × 2,5 in Fig. 6A/C/E; magnification × 10 in Fig. 6B/D/F)

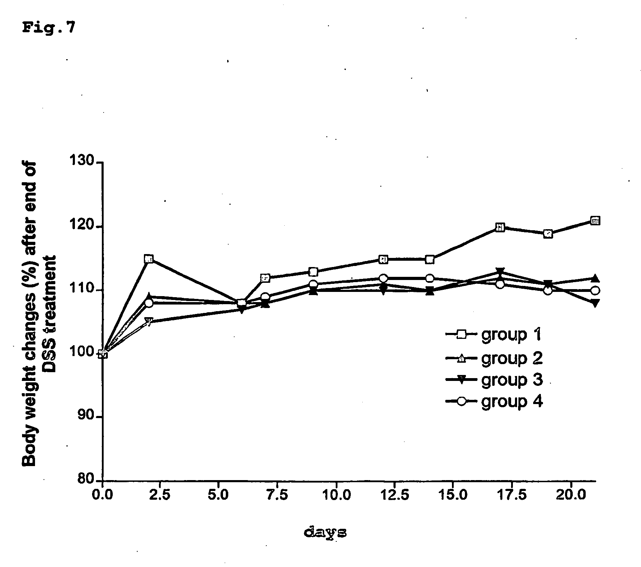

- Figure 7:: Changes in the body weight of mice suffering from dextran sulphate sodium (DSS)-induced chronic colitis in the course of a 3 week period after the end of the DSS treatment. Animals of group 1 (■) have been treated with STIC at day +1, animals of group 2 (▲) have been treated with STIC at day +7, animals of group 3 (V) have been untreated, while animals of group 4 (●) have been treated with "control cells" at day +1. The values are mean values obtained from 5 to 7 mice per group, the standard deviation is always below ± 15 %.

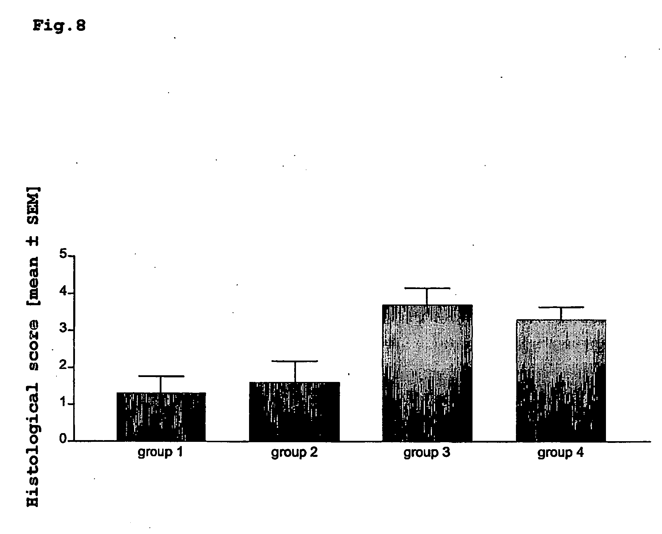

- Figure 8:: Results of the scoring of histochemically stained colon sections of mice suffering from dextran sulphate sodium (DSS)-induced chronic colitis. Animals of group 1 have been treated with STIC at day +1, animals of group 2 have been treated with STIC at day +7, animals of group 3 have been untreated, while animals of group 4 have been treated with "control cells" at day +1. (score 0 = healthy unobtrusive finding; score 1 = minimal colitis; score 2 = moderate colitis; score 3 = heavy colitis; and score 4 = ulcerative colitis accompanied by destruction of the whole mucosa).

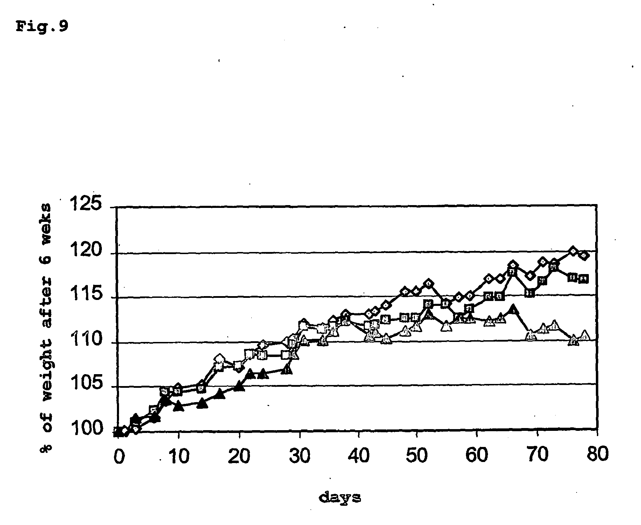

- Figure 9:: Changes in the body weight of mice of the CD62L⁺/CD4⁺ SCID transfer model of chronic experimental colitis with reference to the weight of the mice 6 weeks after the beginning of the experiment when they receive the cell therapy. Animals of group 1 (◆) have been treated with STIC after 6 weeks, animals of group 2 (■) have been untreated and animals of group 3 (▲) have been treated with "control cells" after 6 weeks. The values are mean values obtained from 6 mice per group, the standard deviation is always below ± 15 %.

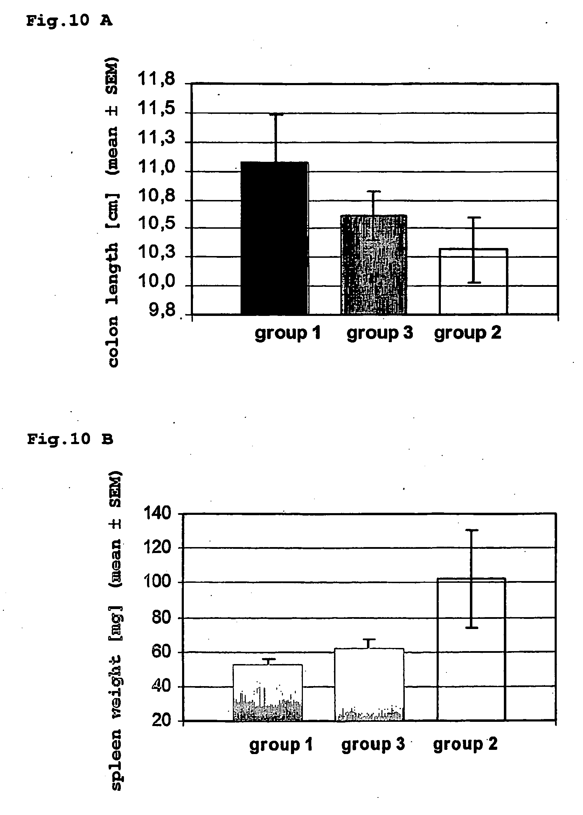

- Figure 10:: Measurement of the colon length (Fig. 10A) and the spleen weight (Fig. 10B) of mice receiving STIC (group 1); of untreated control mice (group 2) and of mice receiving "control cells" (group 3) in the CD62L⁺/CD4⁺ SCID transfer model of chronic experimental colitis. The values are mean values ± SEM.

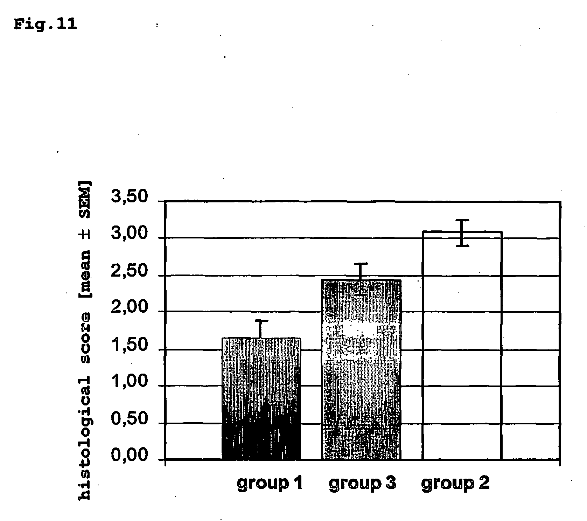

- Figure 11:: Scoring of histochemically stained colon sections of mice of the CD62L⁺/CD4⁺ SCID transfer model of chronic experimental colitis six weeks after cell transfer. Animals of group 1 have received STIC, animals of group 2 have been untreated and have received no cell injection, while animals of group 3 have received "control cells". The values are mean values ± SEM. (score 0 = healthy unobtrusive finding; score 1 = minimal colitis; score 2 = moderate colitis; score 3 = heavy colitis; and score 4 = ulcerative colitis accompanied by destruction of the whole mucosa).

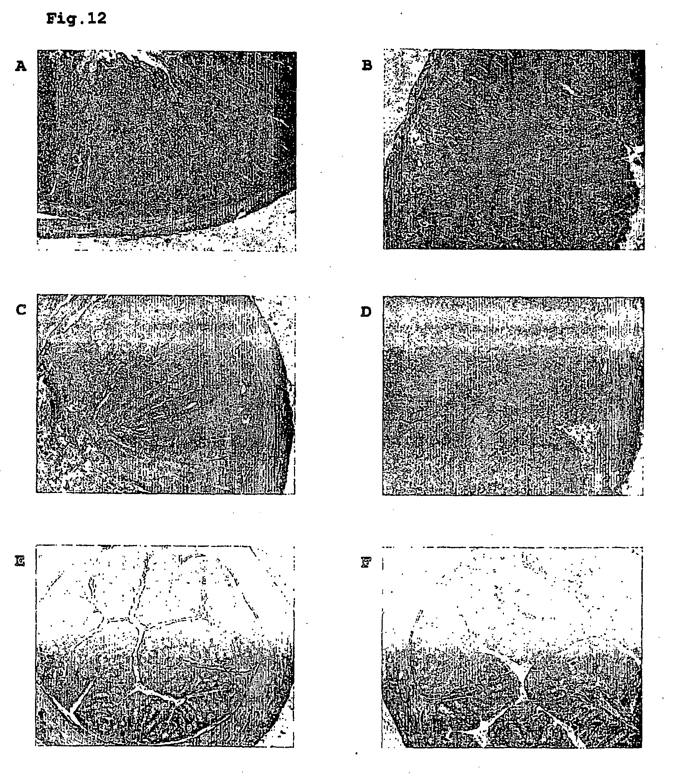

- Figure 12:: H&E staining of colon sections of mice of the CD62L⁺/CD4⁺ SCID transfer model of chronic experimental colitis illustrating the condition of the colon of two untreated animals of group 2 (Fig. 12A/B), of two animals of group 3 treated with "control cells" (Fig. 12C) and of two animals of group 1 treated with STIC (Fig. 12E/F). (magnification × 100)

### Summary of the invention

The basic steps of the process for preparing autologous self-tolerance inducing cells (STIC) of monocytic origin comprise:
(a) Isolating monocytes from the blood of the respective patient to whom the cells are to be administered;
(b) Propagating the monocytes in a suitable culture medium which contains the macrophage colony stimulating factor (hereinafter referred to as M-CSF) as growth promoting agent;
(c) Stimulating the monocytes with γ-interferon (hereinafter called γ-IFN); and
(d) Obtaining the self-tolerance inducing cells formed in stage c) by separating the cells from the culture medium.

A similar process is described in the German Patent Application DE 102 31 655.4 and in the International Patent application WO 2004/007701. In the aforementioned earlier patent applications the cells so prepared are designated "transplant acceptance inducing cells" (TAIC). They are used for inducing acceptance of allogeneic donor tissue in the recipient. Importantly, the TAIC described in DE 102 31 655.4 and in PCT/EP03/07551 are derived from monocytes of the donor and are administered to the recipient in order to induce transplant acceptance. This means that the TAIC are allogeneic to the individual to be treated with the TAIC.

In contrast, the present invention is based on the concept of "autologous treatment"; this means that an individual suffering from disturbed self-tolerance, in particular from autoimmune diseases and/or allergies is treated with self-tolerance inducing cells (STIC), which are derived from autologous monocytes.

Therefore, while TAIC and STIC are both derived from monocytes by essentially the same process, TAIC are allogeneic to the patient to be treated, while STIC are autologous in this respect.

With respect to the process for producing STIC it has been shown that the stimulation with γ-IFN represents a decisive step (see Example 2).

In the context of the present invention, the term self-tolerance inducing cells (STIC) of monocytic origin designates the cell population, which is obtained from step (d) of the process described above. This cell population comprises next to the cells derived from monocytes, which are effective in inducing self-tolerance, also lymphocytes, see Example 4, as well as optionally further cells derived from the mononuclear cell fraction, as for instance granulocytes. The amount of cells derived from monocytes within the STIC-population is preferably 50 to 90 %, more preferably 60 to 70 %, referring to the total cell number.

With respect to the present invention the term "total cell number" refers to the amount of vital cells in the cell population under consideration. This amount can be determined by the "trypan blue dye exclusion technique", since this dye allows to distinguish vital cells from non-vital cells by optical means.

The STIC may usually be used in a quantity of 10⁴ - 10⁶ cells per kilogram body weight, preferably 10⁵ cells per kilogram body weight, to induce self-tolerance. STIC administration may be carried out repeatedly.

The STIC according to the invention have proved to be risk-free regarding the formation of malignoma, both in the animal test and in culture; this is a result which could not have been expected in any other way because of the nature of the original monocytic cell from which the cells according to the invention are derived.

As in detail explained below, further sub-populations of cells having optimized self-tolerance inducing properties can be isolated from the fraction of cells present in STIC, which are derived from monocytes.

Following *in vitro* cultivation and stimulation of the original cells (monocytes) with γ-interferon, STIC are formed, which comprise a sub-population of cells, see Example 3, which binds the monoclonal antibody GM-7, which is expressed by the hybridoma cell line DSM ACC2542. The monoclonal antibody GM-7 is an antibody of the immunoglobulin isotype IgG₂ₐ, the light chain of which exhibits the kappa-isotype. The characteristic property of this antibody is its stringent capacity to bind to the monocytes modified by the culture conditions according to the invention since original monocytic cells are not recognised, i.e. binding of the antibody to the original cells does not take place, (see Example 3). In addition, it was demonstrated with 20 volunteers that GM-7 does not bind to human cells in the peripheral blood, see Figure 5.

As described in WO 2004/007701 the antibody was prepared by immunising mice with TAIC (corresponding to STIC) derived from human monocytes using methods known to the person skilled in the art (Davis, W.C. "Methods in Molecular Biology: Monoclonal Antibody Protocols", New York: Humana Press Inc. Totowa, 1995). A hybridoma cell line was then produced by fusion of a B cell generating the antibody and a myeloma cell from the mouse. Methods used for the preparation of such cell lines are known in the state of the art (Davis, W.C. "Methods in Molecular Biology: Monoclonal Antibody Protocols", New York: Humana Press Inc. Totowa, 1995; Kohler, G., Milstein, C. "Continuous cultures of fused cells secreting antibody of predefined specificity", Nature 256, 495-497 (1975)). The hybridoma cell line producing the antibody GM-7 was deposited according to the rules of the Budapest Convention at DSMZ (Deutsche Sammlung von Mikroorganismen und Zellkultur GmbH, Braunschweig, Germany) under the accession no. DSM ACC2542.

Figure 1 shows the binding capacity, determined by flow cytometry, of GM-7 to monocytic cells after *in vitro* modification according to the invention. It can be seen that the CD14-positive monocytes obtained directly from the mononuclear cell fraction do not bind the antibody GM-7 (the cloud shaded grey is congruent with the non-shaded antibody control). In contrast, following cultivation in the presence of M-CSF and stimulation with γ-IFN, part of the monocytes express an antigen which is recognised by the monoclonal antibody GM-7. The monoclonal antibody GM-7 was characterised as isotype κ-IgG₂ₐ. The process according to the invention consequently leads to a change in the phenotypic pattern of the antigen expression on the cell membrane of the modified monocytes (Figure 1).

The monoclonal antibody GM-7 binds specifically to that cell population which, among those cells produced by the process according to the invention, induce the most effective self-tolerance inducing cells (see Figure 5).

Therefore, a preferred embodiment of the invention relates to such STIC, which are capable of binding the antibody GM-7. These cells are subsequently designated as STIC_{GM7}.

The antibody GM-7 according to the invention therefore represents an extraordinarily effective and easy to handle agent for selecting and purifying the cells inducing self-tolerance (STIC). By means of the antibody, it is possible according to the invention to generate a homogeneous and highly effective STIC population.

According to a preferred embodiment of the invention, the self-tolerance inducing cells formed in step c) of the above described process of the invention, which express the antigen binding to the antibody GM-7, may either be selected directly from the culture medium after step c), or they may be selected from the cell population obtained after separating the cells from the culture medium according to step d) of the above-mentioned process of the invention respectively, by binding to the antibody GM-7 produced by the hybridoma cell line DSM ACC2542.

To select the STIC according to the invention, the antibody is contacted with the sample under conditions which permit binding of the antibody to the self-tolerance inducing cells present in the sample. The reaction complexes resulting from the binding reaction are subsequently separated from the sample. For this purpose, the antibody can be immobilised on a carrier material before contact with the sample; for example, it can be bound to a matrix suitable for chromatographic purposes or to so-called "magnetic beads". This procedure allows to select and concentrate self-tolerance inducing cells from large volumes of sample.

To obtain the self-tolerance inducing cells, the bond between the antibody and the self-tolerance inducing cells is separated after the isolation of the reaction complex from the sample. This can be effected by methods known in the state of the art such as e.g. by competitive displacement or by washing with salt solutions. Corresponding methods are for instance described by Utz U. et al. ("Analysis of the T-cell Receptor repertoire of human T-cell leukemia virus type-1 (HTLV-1) Tax-specific CD8+ Cytotoxic T Lymphocytes from patients with HTLV-1 associated disease: Evidence for the oligoclonal expansion" J. of Virology Feb. 1996, 843-851).

Moreover, the monoclonal antibody GM-7 permits the qualitative and quantitative detection of the self-tolerance inducing cells of monocytic origin according to the invention in blood and/or tissue samples of the patient *in vitro*. The formation of reaction complexes in the sample which indicate the presence and, if applicable, the quantity of the self-tolerance inducing cells is detected by known methods.

To detect the reaction complexes, it is possible in this case to couple ("label") the antibody GM-7, for example, directly with a detectable molecule which is e.g. covalently bound to the antibody. Suitable detectable molecules are described in large numbers in the field of molecular diagnostics and include, among others, fluorescent dyes such as fluorescein isothiocyanate or tetramethyl rhodamine-5-isothiocyanate, luminescent dyes, radioactively labelled molecules and enzymes such as peroxidases (compare Lottspeich, F., Zorbas, H. "Bioanalytik", Spektrum Akademischer Verlag GmbH, Heidelberg-Berlin, 1998).

The detection of the antibody takes place dependent of the molecule selected for labelling of the former. In connection with the present invention, the antibody GM-7 was coupled with the fluorescent molecule fluorescein isothiocyanate (FITC) so that the detection of the antibody could be carried out by means of flow cytometry and/or fluorescence microscopy. Methods for labelling antibodies with FITC are known to the person skilled in the art who operates in the field.

Alternatively, the reaction complex can also be detected in a two-stage process using secondary antibodies. In this connection, the unlabelled antibody GM-7 can be detected in the reaction complex with a further labelled antibody (compare Lottspeich, F., Zorbas, H. "Bioanalytik", Spektrum Akademischer Verlag GmbH, Heidelberg-Berlin, 1998). This two-stage method of detection is considerably more sensitive than the direct detection of binding of the antibody according to the invention since several labelled secondary antibodies can bind to one GM-7 antibody (signal amplification).

The antibody GM-7 consequently allows the detection of STIC in the peripheral blood of the patient treated with STIC, for example in the form of "monitoring", during which the number of cells in the peripheral blood is determined at specific time intervals.

As is obvious to the person skilled in the art, it is possible to prepare monoclonal antibodies against STIC from monocytes also of non-human vertebrates, in particular from monocytes of primates and pigs modified according to the invention. In this respect, the immunisation of the corresponding host animals and the generation of the corresponding hybridoma cell line are carried out as described above for the STIC of human origin.

A particularly preferred embodiment of the invention relates to a sub-population of the STIC of the invention, which co-express the antigens CD3 and CD14 on their cell surface. These cells are subsequently indicated as STIC_{CD3+/CD14+}. As explained in more detail below, these cells have been shown to induce the formation of regulatory T-lymphocytes.

STIC which co-express the surface antigens CD3 and CD14 may either be directly selected from the self-tolerance inducing cells formed in step c) of the above described process of the invention, or they may be selected from the cell population obtained after separating the cells from the culture medium according to step d) of the above-mentioned process of the invention, or they may alternatively be selected from the STIC_{GM7} population.

Further, it has been shown by reference to the TAIC of PCT/EP03/07551 that the cells prepared according to the process described herein express the genes Foxp3, CTLA4 and Integrin α_{E}β₇ strongly (see Example 6, which corresponds to example 12 of PCT/EP03/07551). In contrast, these genes are not or only to a small extent expressed by the original monocytes. The upregulation of the expression of the genes Foxp3, CTLA4 and Integrin α_{E}β₇ is therefore a characteristic of STIC_{CD3+/CD14+}-cells.

As discussed in Example 6, the expression of the markers FoxP3, CTLA4 and Integrin α_{E}β₇ was previously only described for regulatory T-lymphocytes. T-lymphocytes, which co-express the surface antigens CD4 and CD25 are a sub-population of regulatory T-lymphocytes, which are also indicated as "suppressor cells". It is their function to suppress the immune response of the body. In particular, Foxp3 is seen as a specific transcription factor, which serves as a control gene for the development of regulatory T-cells, and which is specifically expressed by these cells. According to the invention, it is preferred that the STIC_{CD3+/CD14+}-cells express at least 1 × 10⁻⁹, more preferably at least 5 × 10⁻⁹, and in particularly preferred manner at least 1 × 10⁻⁸ µg Foxp3-RNA per µg total RNA.

CTLA4 is similarly viewed as a marker for the detection of the regulatory function of T-lymphocytes, in particular of CD4/CD25 positive T-lymphocytes (see the literature cited in Example 6). According to the invention, the STIC_{CD3+/CD14+}-cells should preferably express at least 5 × 10⁻⁷, more preferably at least 3 × 10⁻⁶ and in a particularly preferred manner at least 5 × 10⁻⁶ µg CTLA4-RNA per µg total RNA.

Integrin α_{E}β₇, which recognizes epithelial Cadherin was recently described by Lehmann et al. in PNAS 99, pages 13031-13036 (2002) as a new marker for a sub-population of highly potent regulatory T-lymphocytes, which interact with the epithelial environment. The expression of the Integrin α_{E}β₇-RNA should according to the invention amount in STIC_{CD3+/CD14+}-cells to preferably at least 1 × 10⁻¹², more preferably to at least 1 × 10⁻¹¹, and in a particularly preferred manner to at least 1 × 10⁻¹⁰, and most preferably to at least 1 × 10⁻⁹ µg per 1 µg total RNA.

As shown in the Table in Example 6, the direct co-culturing of the cells of the invention with lymphocytes leads to a significant increase in the number of regulatory T-lymphocytes, in particular of CD4⁺/CD25⁺ cells in the lymphocyte population with strongly up-regulated expression of the genes Foxp3, CTLA4 and Integrin α_{E}β₇. The Example further demonstrates that this effect is not observed if the cells of the invention are indirectly co-cultured with lymphocytes.

These results indicate that stimulation of the formation and/or propagation of regulatory T-lymphocytes by the cells of the invention is involved in the induction of self-tolerance by these cells.

Example 7 (corresponds to Example 13 of PCT/EP03/ß7551) confirms the hypothesis of an involvement of the cells of the invention in the induction of an immune response suppression by reference to the TAIC of PCT/EP03/07551 (see above). In this Example, lymphocytes from recipient animals were incubated with immune suppressive cells from the respective donor animals *in vitro.* For the induction of tolerance, lymphocytes from the recipient pre-incubated with donor derived TAIC were injected to the animals instead of TAIC. Donor specific tolerance could be induced also in this manner, while animals, to which recipient lymphocytes not co-cultivated with donor derived TAIC were administered, did not develop tolerance.

The STIC of the invention can be used as such as a pharmaceutical preparation. The cells obtained from step d) of the method of the invention as described above can be used directly. About 10 - 50 % of the total cells of the populations so obtained is formed by lymphocytes and granulocytes, which stem from the initial monocyte isolate (mononuclear cell fraction). These cells support the formation of the STIC of the invention derived from the monocytes in the culturing step (see Example 4); they do not interfere with the induction of self-tolerance if the STIC of the invention are used as a pharmaceutical preparation.

However, according to further preferred embodiments of the invention, the sub-populations STIC_{GM7} and/or STIC_{CD3+/CD14+} may be isolated from the totality of the STIC population obtained from process of the invention (see above) and may be used for self-tolerance induction.

In culture media (see Example 2), the STIC and/or the STIC_{GM7} and/or STIC_{CD3+/CD14+} may be kept for at least 48 hours without their self-tolerance inducing effect becoming lost.

For use as pharmaceutical preparation, the STIC and/or the subpopulations STIC_{GM-7} and/or STIC_{CD3+/CD14+} suspended in e.g. human AB0 compatible serum (universally suitable for use) can be administered intravenously as short transfusion.

In this context pharmaceutical preparations may comprise the STIC of the invention in combination with conventional antiinflammatory agents and/or conventional immunosuppressants such as steroids, in particular cortisone, methotrexate, cyclophosphamide, azathioprine, 5-aminosalicylic acid (5-ASA), TNF-α antibodies, α-interferon and B cell antibodies, such as e.g. Rituximab, for the treatment of organ specific or systemic autoimmune diseases.

Further, the STIC of the invention may be used for the treatment of allergies in combination with antihistaminics, theophylline preparations, ß-mimetics, steroids, in particular cortisone, and chromoglycine acid.

### Detailed description of the invention

The starting cells for the process according to the invention are autologous blood monocytes, i.e. monocytes from the blood of the respective patient to whom the cells of the invention are to be administered. Preferably, the autologous monocytes are from human blood. The monocytes may be obtained by any isolation process, in particular by leukapheresis or from the mononuclear cell fraction from whole blood (buffy coat). Leukapheresis is particularly preferred.

Leukapheresis is a general term for a multi-step procedure using a commercially available apheresis apparatus, wherein whole blood from a human subject is withdrawn, separated into fractions and the fractions, except for the mononuclear cell fraction, are retransfused into the human subject. This procedure may be carried out as outlined e.g. in further detail in EP 0 591 194 B1.

Alternatively, the blood can first be separated, after the usual treatment with an anticoagulant, into plasma and into white and red blood cells using methods known in the art, preferably by centrifugation. After centrifugation, the plasma will be present in the supernatant; below it, there is a layer which contains the white blood cells in their entirety. This layer is also referred to as buffy-coat. Below this is the phase containing the red blood cells (hematocrit).

In connection with the process according to the invention, the mononuclear cell fraction is first isolated and separated to obtain the monocytes e.g. by centrifuging according to known methods. According to a preferred process embodiment, the mononuclear cell fraction is applied onto a lymphocyte separation medium (Ficoll-Hypaque) and centrifuged (see Example 1). Example 1 describes the preferred embodiment of the invention, wherein the erythrocytes and dead cells which might still be contained in the mononuclear cell fraction are separated by centrifuging, and the white blood cells including the monocytes are present as an isolate on the separation medium. Thereafter, the white phase may be carefully pipetted off, and, for enrichment of the monocytes within the isolate, is repeatedly centrifuged and washed. In the course of this process, the monocytes will assemble at the bottom of the centrifuge vessel together with a part of the lymphocytes.

According to a particularly preferred embodiment of the method of the invention, the conditions for obtaining the monocyte containing isolate are controlled such that the isolate contains about 10 - 50 % lymphocytes next to the monocytes, by reference to the total number of cells. Preferably, the isolate contains about 50 - 90 %, in a particularly preferred manner 60 - 70 % monocytes and about 10 - 50 %, in a particularly preferred manner 20 - 50 % lymphocytes, each by reference to the total cell count, wherein the difference will optionally be provided by granolocytes.

As shown in Example 4, the presence of lymphocytes in a magnitude of 20 - 30 %, referred to the total cell count, during the culturing of the original monocytes with MCSF and γ-interferon will lead to the generation of a significantly higher amount of CD3/CD14-double positive STIC, as will be the case if only few lymphocytes (about 5 %) are present (see Figure 3).

To prepare a sufficient quantity of STIC it is first necessary to allow propagation of the monocytes. For this purpose, known growth media suitable for monocytes can be used; however, the medium must contain the growth factor M-CSF (macrophage-colony-stimulating-factor). M-CSF (also-called CSF-1) is produced by monocytes, fibroblasts, lymphocytes and endothelial cells. The concentration of M-CSF in the culture medium may preferably amount from 2 to 20 µg/l medium, more preferably 4 to 6 µg/l and in particularly preferred manner 5 µg/l.

Preferably, the growth medium does not contain the granulocyte macrophage colony stimulating factor (GM-CSF), because the yield of STIC is reduced in the presence of this factor.

Subsequently or simultaneously, the cells must be stimulated with γ-IFN, i.e. they must be cultured in the presence of γ-IFN. The stimulation of the monocytes with γ-IFN takes place after an initial propagation phase lasting 3 to 6 days in the culture medium containing the growth factor. Preferably on day 4 after the beginning of cultivation in the presence of M-CSF, γ-IFN stimulation is started and this stimulation is extended over a period of preferably 24 to 72 hours, more preferably 48 hours under incubator conditions i.e. at 37°C and in a 5% CO₂ atmosphere.

The concentration of γ-IFN in the medium may be 0.1 to 20 ng/ml, preferably 1 to 10 ng/ml and particularly preferably 5 ng/ml.

The stimulation with γ-IFN may begin simultaneously with the propagation of the monocytes in the medium containing the growth factor. However, stimulation after a 3 to 6 day long initial propagation phase, as indicated above, is preferred. The propagation of the cells and stimulation with γ-IFN should, overall, preferably not take more than 8 days. In any case, treatment with γ-IFN should be carried out such that after the propagation phase it lasts for at least 24 hours, at maximum 72 hours, preferably 48 hours. The period for propagation and stimulation of the cells should consequently last for a total of preferably 4 to 8 days.

According to a preferred embodiment of the invention, the propagation and stimulation with γ-interferon is carried out as indicated in Example 2 in such a way that the monocytes are first propagated in a culture medium containing the growth factor and that γ-IFN is added to the culture medium after 3 to 6 days in such an amount that a concentration of 0.1 to 20 ng/ml, preferably 1 to 10 ng/ml and particularly preferably 5 ng/ml is obtained in the medium.

Preferably, the process according to the invention is carried out in culture vessels the surface of which has previously been coated with fetal calf serum (FCS) or preferably with human AB0 compatible serum (see Example 2). Coating with FCS can take place by covering the surface of the culture vessels with FCS before use and, following a period of interaction of a few hours, in particular 4 to 72 hours, preferably 12 to 48 hours and in particular 24 hours, removing the FCS not adhering to the surface in a suitable manner. The coating with human AB0 compatible serum is carried out in the same manner as described above.

During the culturing step the cells will settle at the bottom of the culturing vessel after about 24 hours. Due to their adhesive properties the monocytes and the STIC, derived from the monocytes during the process, will then adhere to the bottom of the respective culture vessel. If, as described in Example 2, the culture medium is changed during cultivation, the supernatant is initially carefully removed, for instance by pipetting off or decanting, and subsequently fresh culture medium is filled in. Preferably, however, the cells adhering to the bottom are not or only carefully washed, so that any lymphocytes present are not removed.

Removing the adhering cells can take place mechanically, e.g. by means of a fine cell scraper or spatula.

According to a preferred embodiment of the process according to the invention, however, the complete removal of the cell takes place by treatment with a suitable enzyme, e.g. with trypsin (see Example 2). The trypsin solution (0.1 to 0.025 g/l, preferably 0.05 g/l) can be allowed to act onto the cells for 2 to 10 minutes at 35°C to 39°C, preferably at 37°C, in the presence of 5% CO₂.

The enzyme activity is then blocked in the usual manner and the now freely floating STIC can be obtained in the usual way by centrifuging. They are then available for immediate use, optionally in suspension in a suitable medium, e.g. in PBS. However, they can also be kept for several days, in particular for approximately 2 to 3 days, in a nutrient medium (see Example 2), wherein this preservation medium should contain neither the growth factor nor γ-IFN. The cells can be kept in such a nutrient medium as STIC for at least 48 hours.

For storage over longer periods, the cells can be deep frozen. Protocols for deep freezing of living cells are known in the state of the art, compare Griffith M. et al., ("Epithelial Cell Culture, Cornea", in Methods of tissue engineering, Atala A. and Lanza R.P., Academic Press 2002, chapter 4, pages 131-140). A preferred suspension medium for deep freezing of the cells according to the invention is AB0 compatible serum or FCS both supplemented with DMSO.

According to one embodiment of the invention the cell suspension containing self-tolerance inducing cells obtained from steps c) or d) may be further purified with respect to those cells, which bind the antibody GM-7, so as to obtain a sub-population STIC_{GM7}. Methods for such purification are described above in detail.

According to a further preferred embodiment of the invention such cells are selected from the STIC population, which co-express the antigens CD3 and CD14 on their cell surfaces. Methods for selecting such cells are known in the art. Examples for such methods are the "Fluorescent-Activating Cell Sorting" (FACS), the "Immuno Magnetic Bead Sorting" and the "Magnetic Activated Cell Sorting" (MACS), or the so-called "Rosetting Method" [see Gmelig-Meyling F. et al. "Simplified procedure for the separation of human T- and non-T-cells", Vox Sang. 33, 5-8 (1977)].

The selection of the STIC sub-population STIC_{CD3+/CD14+} can take place directly from the STIC population obtained from steps c) or d) of the above-described process of the invention, or from the STIC_{CM7}-sub-population. The latter way to proceed means that a stepwise enrichment of STIC_{CD3+/CD14+} will take place.

In a preferred embodiment of the invention the cells of the STIC_{CD3+/CD14+} sub-population according to the invention are used per se for the production of a pharmaceutical composition for the *in vivo* prevention and/or treatment of autoimmune diseases.

Examples of such autoimmune diseases are:
- Rheumatic diseases with autoimmune features, in particular rheumatoid arthritis, SLE, Sjögren's disease, scleroderma, dermatomyositis, polymyositis, Reiter's syndrome;
- Diabetes mellitus;
- Autoimmune diseases of the blood and blood vessels, in particular autoimmune hemolytic anemia (AIHA), autoimmune thrombocytopenic purpura (ITP), anti-phospholipid antibody syndrome, vasculitis (Polyarteritis nodosa group, Wegener's granulomatosis, hypersensitivity vasculitis, giant cell arteritis), systemic lupus erythematosus, mixed essential cyroglobulinemia;
- Autoimmune diseases of the liver, in particular autoimmune hepatitis, primary biliary cirrhosis and primary sclerosing cholangitis;
- Autoimmune diseases of the thyroid, in particular Hashimoto's disease, Graves' disease;
- Autoimmune diseases of the central nervous system, in particular multiple sclerosis, myasthenia gravis; and
- Bullous Skin Diseases, in particular pemphigus vulgaris, pemphigus vegetans, pemphigus foliaceus, Senear-Usher syndrome and Brazilian pemphigus.

The usefulness of STIC for inducing self-tolerance is shown for mice in two model systems of artificially induced colitis resembling an autoimmune disease (see Examples 8 and 9).

These models are the dextran sulphate sodium (DSS)-induced chronic colitis (Example 8) and the CD62L⁺/CD4⁺ SCID transfer model of chronic experimental colitis in mice (Example 9). In both models mice develop a chronic colitis with symptoms resembling the human colitis ulcerosa.

In both model systems administration of STIC shortly after the disease onset leads to the suppression of the typical symptoms of the colitis compared to untreated animals or animals treated with "control cells" that do not contain STIC (see Examples 8 and 9).

As discussed above, excessive immune reactions are also involved in the development of allergies. It has been shown by Bach et al. (loc. cit.), that CD4⁺ cells, which comprise CD4⁺/CD25⁺ cells as a subpopulation, can mitigate the progression of experimental allergic encephalomyelitis (EAE). Since, as shown herein, the administration of STIC results in the induction of an increase of the CD4⁺/CD25⁺ T cell population, the cells are also useful for the treatment of allergies.

So, according to a further preferred embodiment of the invention, STIC containing pharmaceuticals are used for the prevention and/or treatment of allergies.

Pharmaceutical preparations may contain vital STIC according to the invention, which are obtained from step d) of the process of the invention, suspended in a pharmaceutically acceptable carrier preferably in a quantity of about 1 × 10⁵ to 1 × 10⁷ cells/ml and more preferably of about 1 × 10⁶ cells/ml of the preparation.

In a further preferred embodiment of the invention the cells of the STIC_{GM7} sub-population according to the invention are used per se for the production of a pharmaceutical composition for the *in vivo* prevention and/or treatment of diseases associated with disturbed self-tolerance.

In one embodiment of the invention such pharmaceutical preparation may contain vital STIC_{GM7} cells according to the invention, which bind to the antibody GM-7, suspended in a pharmacologically acceptable liquid carrier, preferably in a quantity of about 1 × 10⁶ to 1 × 10⁸ cells/ml and more preferably of about 1 × 10⁶ cells/ml of the preparation.

In the most preferred embodiment of the invention such pharmaceutical preparation may contain vital STIC_{CD3+/CD14+} cells according to the invention, which co-express the antigens CD3 an CD14, in a quantity of preferably about 5 × 10⁵ to 5 × 10⁷ cells/ml and more preferably of about 5 × 10⁶ cells/ml of the preparation.

The above described pharmaceutical preparations may contain the cells according to the invention suspended in a physiologically well-tolerated medium. Suitable media are for example Ringer solution, physiological saline or 5 to 20 % human albumin solution and the like.

The pharmaceutical compositions containing STIC according to the invention may be administered via a variety of routes of administration depending on the body site(s) affected by the respective disease. In preferred embodiments of the invention said pharmaceutical compositions can be administered intravenously, intraportally, subcutaneously, intradermally, intraperitoneally or intrathecally. The pharmaceutical compositions can further be administered directly into a specific organ, such as the liver or muscle, per inhalation or into body cavities.

The pharmaceutical preparations containing STIC as active ingredients can also be used for the prevention of autoimmune diseases in those cases where the gene responsible for the development of the disease is known. If an individual is diagnosed as a carrier of a gene associated with one or more autoimmune diseases, the outbreak of the disease can be prevented by administering STIC derived from autologous monocytes in an early stage of life. In a preferred embodiment of the invention the autologous STIC are cocultured with autologous lymphocytes and with the peptide expressed by the respective gene. This will lead to the development of autologous regulatory T-lymphocytes specific for the respective gene product. These regulatory T-lymphocytes can then be re-administered to the respective individual as described below in Example 7 (corresponds to Example 13 of WO 2004/007701).

In a further embodiment of the invention, STIC can be used for prevention in those cases where the autoimmune disease occurs in intervals, as for instance in rheumatoid arthritis. If the pharmaceutical preparation containing STIC is administered after the first or a subsequent break out of the disease, further break outs can be prevented.

Cell preparations according to the invention may contain vital STIC, which are obtained from step d) of the process of the invention. Alternatively, the preparations may contain cells belonging to the sub-populations of STIC_{GM7} cells, which bind to the antibody GM-7, or of STIC_{CD3+/CD14+} cells, which co-express the antigens CD3 and CD14 on their cell surface. The preparations may contain the respective cells in a quantity of preferably at least 1 × 10⁵, more preferably at least 5 × 10⁵ and most preferably at least 1 × 10⁶ cells per ml suspended in a liquid carrier medium. The medium may be a cell culture or transport medium well-tolerated by cells, such as 5 to 20% human albumin solution. Alternatively, the cells within the preparation may be deep-frozen and contained in a suitable storage medium, such as e.g. RPMI with 50% human albumin solution and 10% DMSO.

Finally, the invention also relates to a method wherein the self-tolerance inducing cells of the invention (STIC, STIC_{GM7} or STIC_{CD3+/CD14+}) are used for generating or expanding autologous regulatory T-lymphocytes *in vitro.* As shown in Example 6 (corresponding to Example 12 of WO 2004/007701), the direct *in vitro* cocultivation of TAIC, corresponding to STIC with lymphocytes leads to a significant proliferation of regulatory T-lymphocytes, in particular of CD4⁺/CD25⁺ lymphocytes. It is therefore possible to generate and/or expand autologous regulatory T-lymphocytes, in particular CD4⁺/CD25⁺ lymphocytes, by directly coculturing STIC derived from monocytes of an individual with autologous lymphocytes.

Direct *in vitro* culturing means according to the invention that STIC and lymphocytes are cocultured in direct physical contact within the same medium, as l.c. exemplified in Example 6.

In this method the medium preferably contains the respective cells i.e. STIC and lymphocytes in about equal cell numbers and each in a quantity of preferably at least 1 × 10⁵, more preferably at least 5 × 10⁵ and most preferably at least 1 × 10⁶ cells per ml suspended in a liquid carrier medium; the medium may be a cell culture or transport medium well-tolerated by cells, such as 5 to 20% human albumin solution. The co-cultivation preferably should take place under physiological conditions at about 37°C, e.g. in an incubator, for preferably about 3 to 5, more preferably 4 days.

It was shown in Example 7 (corresponding to Example 13 of WO 2004/007701), that transplant acceptance can not only be induced by administering TAIC generated from donor monocytes to the recipient, but also by readministering recipient lymphocytes to the recipient, which have previously been directly cocultured *in vitro* with TAIC prepared from donor monocytes as described above. Similarly, self-tolerance can be induced by readministering autologous lymphocytes to the patient, which have previously been directly co-cultured with STIC prepared from the patient's own monocytes.

According to a further embodiment of the invention regulatory T-lymphocytes can therefore be prepared *in vitro* from lymphocytes originating from the individual to be treated, by direct coculturing of lymphocytes from this individual with STIC originating from monocytes of this individual. Readministering the cocultivated lymphocytes to the recipient will lead to induction of self-tolerance, as shown in Example 7.

The regulatory T-lymphocytes so prepared may be isolated by FACS as described herein (see above) and may be used in a pharmaceutical preparation for preventing and/or treating of diseases associated with disturbed self-tolerance wherein the cells are suspended in a pharmacologically acceptable carrier as described above.

The invention is illustrated in further detail by way of examples.

If not defined within the examples, the composition of the media and substances used are as follows:
**1. Penicillin/streptomycin solution:**
10,000 units of penicillin as sodium salt of penicillin G and 1000 µg streptomycin as streptomycin sulphate per ml physiological sodium chloride solution (NaCl 0,85 %) (Gibco Catalogue No. 15140122).
**2. Trypsin-EDTA**
0.5 g trypsin and 0.2 g EDTA (4 Na)/l
**3. RPMI 1640 (1x, liquid (11875))**
**contains L-Glutamine**
RPMI (Roswell Park Memorial Institute) Media 1640 are enriched formulations, which can be used extensively for mammalian cells.

| **Components** | **Mol.-weight** | **Conc. (mg/I)** | **Molarity (nM)** |
|---|---|---|---|
| **Inorganic salts** | | | |
| Calcium nitrate (Ca(NO₃)₂ 4H₂O) | 236 | 100.00 | 0.424 |
| Potassium chloride (KCl) | 75 | 400.00 | 5.30 |
| Magnesium sulphate (MgSO₄) | 120 | 48.84 | 0.407 |
| Sodium chloride (NaCl) | 58 | 6000.00 | 103.44 |
| Sodium bicarbonate (NaHCO₃) | 84 | 2000.00 | 23.800 |
| Sodium phosphate (Na₂HPO₄) | 142 | 800.00 | 5.63 |
| **Further components** | | | |
| Glucose | 180 | 2000.00 | 11.10 |
| Glutathione, reduced | 307 | 1.50 | 0.0032 |
| Phenol red | 398 | 5.00 | 0.0125 |
| **Amino acids** | | | |
| L-Arginine | 174 | 200.00 | 1.10 |
| L-Asparagine | 132 | 50.00 | 0.379 |
| L-Asparaginic acid | 133 | 20.00 | 0.150 |
| L-Cysteine dihydrochloride | 313 | 65.00 | 0.206 |
| L-Glutamininc acid | 147 | 20.00 | 0.136 |
| L-Glutamine | 146 | 300.00 | 2.05 |
| Glycine | 75 | 10.00 | 0.133 |
| L-Histidine | 155 | 15.00 | 0.0967 |
| L-Hydroxyproline | 131 | 20.00 | 0.153 |
| L-Isoleucine | 131 | 50.00 | 0.382 |
| L-Leucine | 131 | 50.00 | 0.382 |
| L-Lysine hydrochloride | 146 | 40.00 | 0.219 |
| L-Methionine | 149 | 15.00 | 0.101 |
| L-Phenylalanine | 165 | 15.00 | 0.0909 |
| L-Proline | 115 | 20.00 | 0.174 |
| L-Serine | 105 | 30.00 | 0.286 |
| L-Threonine | 119 | 20.00 | 0.168 |
| L-Tryptophan | 204 | 5.00 | 0.0245 |
| L-Tyrosine disodium, dihydrate | 261 | 29.00 | 0.110 |
| L-Valine | 117 | 20.00 | 0.171 |
| **Vitamins** | | | |
| Biotin | 244 | 0.20 | 0.008 |
| D-calcium pantothenate | 477 | 0.25 | 0.0005 |
| Choline chloride | 140 | 3.00 | 0.0214 |
| Folic acid | 441 | 1.00 | 0.0022 |
| i-Inositol | 180 | 35.00 | 0.194 |
| Niacinamide | 122 | 1.00 | 0.0081 |
| p-aminobenzoic acid (PABA) | 137 | 1.00 | 0.0072 |
| Pyridoxine HCl | 206 | 1.00 | 0.0048 |
| Riboflavin | 376 | 0.20 | 0.0005 |
| Thiamin HCl | 337 | 1.00 | 0.0029 |
| Vitamin B12 | 1355 | 0.005 | 0.00000369 |

Reference: Moore G.E., et al., J.A.M.A. 199: 519 (1967)
**4. PBS (Dulbecco's phosphate buffered saline) cf.** J. Exp. Med. 98:167 (1954):

| Components | g/l |
|---|---|
| KCl | 0.2 |
| KH₂PO₄ | 0.2 |
| NaCl | 8.00 |
| Na₂PHO₄ | 1.15 |

**5. Ficoll-Hypaque:**
Lymphocyte separation medium (saccharose/epichlorohydrincopolymerisate Mg 400,000; Density 1.077, adjusted with Sodium diatrizoate).

**6. L-Glutamine**
Liquid: 29.2 mg/ml
**7. Macrophage-colony stimulating factor (M-CSF)**
Recombinant human M-CSF from E. coli; contains as monomer (18.5 kD) 135 amino acid residues including the N-terminal methionine; is present as a homodimer with a molar mass of 37 kD; (SIGMA Catalogue No. M 6518)
**8.** γ**-Interferon (**γ**-IFN)**
Recombinant human γ-IFN from E. coli; 16.7 kD protein containing 143 amino acid residues (CHEMICON Catalogue No. IF002)
**9. Dextran sulphate sodium**
Sigma-Aldrich 31403, salt, MW 40 kD

### Example 1

### Separation of Monocytes from whole Blood

Whole blood was obtained from human patients by two different methods:
a) Leukapheresis: Leukapheresis was carried out using a COBE^{®} Spectra^{™} apheresis system (Gambro BCT, Lakewood, CO, USA) in the mononuclear mode (MNZ) according to the manufacturer's instructions (COBE Spectra Version 4.7/5.1/6.0/7.0).
b) Conventional separation of blood components: 450 ml whole blood was mixed in a triple chamber bag set with 63 ml of a stabiliser solution which contained, per litre H₂O 3.27 g of citric acid, 26.3 of g trisodium citrate, 25.5 g of dextrose and 22.22 g of sodium dihydroxy phosphate to avoid coagulation of the blood and to feed the cells. The pH of the solution was 5.6-5.8.
   To separate the components of the blood, "sharp centrifuging" of this mixture was subsequently carried out at 4000 rpm for 7 minutes at 20°C. This resulted in the layering of the corpuscular and non-corpuscular components within three layers. By using the bag set in a compression machine provided for this purpose, the erythrocytes were then pressed into the lower bag, the plasma was pressed into the upper bag and the so-called buffy-coat remained in the middle bag and contained a volume of approximately 50 ml.
   From both methods, the quantity of 50 ml of freshly obtained mononuclear cell fraction was then divided into 2 portions of 25 ml each and layered onto 25 ml of Ficoll-Hypaque separation medium respectively which had been introduced previously into two 50 ml Falcon tubes.
   This preparation was centrifuged for 30 minutes at 2500 rpm without braking. Thereafter, any erythrocytes and dead cells that might still be present in the mononuclear cell fraction were below the Ficoll phase, whereas the white blood cells, including the monocytes, were separated off on the Ficoll as white interphase.
   Subsequently, the white interphase including the monocytes was carefully removed by pipetting and mixed with 10 ml of phosphate-buffered saline (PBS).
   This preparation was then centrifuged three times for 10 minutes at 1800 rpm with braking, the supernatant being removed by pipetting after each centrifuging process and fresh PBS being introduced.
   The cell pellets assembled at the bottom of the centrifuging vessel (Falcon tubes) contained the mononuclear cell fraction, i.e. the monocytes.
c) The monocytes required for the mouse experiments 8 and 9 (performed with genetically identical mice) were obtained from spleens of syngeneic donor mice. The spleens were passed through a small meshed sieve under slight pressure and the thus separated cells were resuspended in PBS. The suspended spleen cells were layered onto a 25 ml Ficoll-Hypaque separation medium which had been introduced previously into 50 ml Falcon tubes. The procedure was then continued in the same way as described above.

### Example 2

### Propagation and Modification of the Monocytes

The cultivation and propagation of the monocytes was carried out in nutrient medium with the following composition:

| | |
|---|---|
| RPMI 1640 medium | 440 ml |
| Fetal calf serum (FCS), alternatively, AB0 compatible serum | 50 ml |
| Penicillin / Streptomycin Solution | 5 ml |
| Total volume | 500 ml |

The nutrient medium contained 2,5 µg / 500 ml M-CSF.

The monocytes isolated in Example 1 were suspended in a total quantity of 10⁶ cells in 10 ml of the nutrient medium and transferred onto a Petri dish (100 mm in diameter). The Petri dish had previously been filled with pure inactivated FCS and the FCS had been poured off after 24 hours in order to obtain, in this way, a dish coated with FCS.

The Petri dish was covered with the appropriate cover and kept for 3 days in an incubator at 37°C. The cells settled at the bottom of the Petri dish after 24 hours. On day two, the supernatant was pipetted off and the Petri dish again filled with 10 ml of fresh nutrient medium.

On day 4, 50 ng γ-interferon in 10 ml nutrient medium were added and the dish was again closed and kept for a further 48 hours in the incubator at 37°C.

Subsequently, 10 ml of trypsin solution each diluted 1:10 with PBS were pipetted into the Petri dish. The closed Petri dish was kept for 10 minutes in the incubator at 37°C.

Thereafter, the separation of the cells adhering to the bottom of the Petri dish was carried out with a cell scraper in such a way that the major part (>90%) of the cells floated in the supernatant.

The total supernatant (10 ml of trypsin solution + 10 ml of medium) was pipetted off, combined in a 50 ml Falcon tube and centrifuged for 10 minutes at 1800 rpm. The supernatant was then discarded and fresh nutrient medium (see above) was added to the precipitate (the remaining cell pellet), 1 ml of nutrient being added per 10⁶ cells. The determination of the cell count for the determination of the exact dosage took place according to known methods, compare Hay R.J.; "Cell Quantification and Characterisation", in Methods of Tissue Engineering, Academic Press 2002, Chapter 4, p. 55-84.

This cell suspension was centrifuged (1800 rpm, 10 minutes, see above) and the cell pellet were incorporated either into PBS or for application in man into NaCl (physiol.). Subsequently, the intravenous administration can take place directly or within 48 hours.

Alternatively, after centrifuging and discarding the supernatant containing trypsin, FCS/DMSO was added as freezing medium to the cells and these were deep frozen in a volume of 10 ml.

The freezing medium contained 95% FCS and 5% DMSO. In each case, approximately 10⁶ cells were incorporated into 1 ml of the medium and cooled in the following steps:
30 minutes on ice;
2 hours at -20°C in the pre-cooled Styropor box;
24 hours at -80°C in Styropor;
Storage in small tubes in liquid nitrogen (N₂) at -180°C.

Figure 1 shows the phenotypic changes in the antigen expression, determined by flow cytometry, on the monocytes used after cultivation and γ-IFN stimulation of the original monocytic cells. The cultivation and γ-IFN stimulation of the original monocytic cells leads to significantly increased binding of GM-7 after modification (right hand graph in Fig. 1) compared to cells before modification (left hand graph in Fig. 1).

Examples 3 to 7 below are taken from WO 2004/007701. They characterise the transplant-acceptance inducing cells (TAIC) disclosed therein. Due to the similarity of the TAIC of PCT/EP03/07551 and the self-tolerance inducing cells (STIC) of the present invention (see above) the results reported in these examples also apply to the STIC.

More precisely, the results of Examples 3 to 7 below show
■ that a cell population with optimised suppressor function can be isolated from the TAIC population obtained after cultivation of original monocytes with M-CSF and γ-IFN-stimulation through the availability of the monoclonal antibody GM-7 (Example 3):
■ that lymphocytes present in the monocyte fraction have a great influence on the generation of CD14⁺/CD3⁺-cells effective as TAIC (Example 4);
■ that the IDO-inhibitor 1-methyltryptophane has no influence on the suppressor function of the TAIC (Example 5);
■ that direct *in vitro* co-culture of TAIC of donor A together with lymphocytes of a donor B. induces the formation of regulatory T-cells, i.e. CD4⁺/CD25⁺-lymphocytes (Example 6); and
■ that the physical cell to cell interaction between donor-TAIC and recipient-lymphocytes *in vivo* induces the generation of regulatory T-cells capable of preventing organ rejection (Example 7).

### Example 3

### Binding of the Antibody GM-7 to TAIC

The monoclonal antibody GM-7 was generated by immunising mice with human TAIC which had been prepared as described in PCT/EP03/07551. The hybridoma cells producing the antibody were deposited at the "Deutsche Sammlung für Mikroorganismen" under the accession no. DSM ACC2542. The results reported below demonstrate that the antibody binds specifically to an antigen which is expressed only on CD14⁺ cells which had been subjected to the 6 day ex situ modification with M-CSF and 2 day γ-IFN stimulation according to the invention.

Figure 1 shows the binding capacity, determined by flow cytometry, of GM-7 to monocytic cells after *in vitro* modification, i.e. after transformation into TAIC. It can be seen that the CD14-positive monocytes obtained directly from buffy-coat do not bind the antibody GM-7 (left-hand picture; the grey shaded cloud corresponds to the antibody control). In contrast, part of the monocytes express an antigen after cultivation in M-CSF and stimulation with γ-IFN, which antigen is recognised by the monoclonal antibody GM-7. After cultivation as described in Example 2, about 80% of the transformed monocytes are able to bind the monoclonal antibody GM-7 (righthand picture) .

In a further experiment, the suppressor activity of the CD14⁺/GM-7⁺ cells was compared with that of CD14⁺/GM-7⁻ cells in a mixed lymphocyte culture (MLC). The MLC was carried out as described by Kurnick, J.T. "Cellular Assays" in: Diagnostic Immunopathology, [Colvin R.B., Bhan A.K., McCluskey, R.U. (ed.), Raven Press, New York, pages 751-771 (1994)].

In this example, the TAIC stem from an individual B. As shown in Figure 2, a significant difference exist between the suppressor activity of the GM-7 positive and GM-7 negative TAIC. Only the GM-7 positive fraction the TAIC population obtained after 6 days treatment with M-CSF and 2 days stimulation with γ-IFN exhibits a significant inhibition effect on the T-cell proliferation activity of responder cells of the individual A after stimulation with cells of B.

### Example 4

### Influence of Lymphocytes on the Formation of CD3⁺/CD14⁺-Cells during Monocyte Cultivation

The influence of lymphocytes present in the monocyte fraction on the generation of CD14⁺/CD3⁺-cells effective as TAIC was determined by comparison of two different set ups.

For the first set up (subsequently indicated as "Mo") the monocyte fraction was initially taken from the interphase of the buffy-coat as described in Example 1. As described in Example 2, the cells were subsequently transferred to the cultivation step with M-CSF. Within one hour after the starting point of the cultivation, the monocytes adhering to the bottom of the tissue culture flask were washed five times with 10 ml PBS each, and the amount of the lymphocytes present in the culture was thereby decreased to < 5 % (4.8 ± 2.4 %), while the amount of enriched monocytes (CD14⁺) so obtained was above 90 % (92 ± 5.6 %). Additional cell components within the set up were B-lymphocytes and granulocytes.

The cells in the second set up (subsequently indicated as "Mo+Ly") were also taken from the interphase of the buffy-coat as monocyte fraction as described in Example 1. However, different from set up "Mo" the cells adhering to the bottom of the tissue culture flask were only washed once after 24 hours starting from the beginning of the cultivation phase, as described in Example 2. As a result, a cell population was obtained, which was composed of 45 ± 5.3 % CD14⁺-monocytes and 23.5 ± 8.9 % CD2⁺-lymphocytes. As in set up "Mo" lymphocytes and granulocytes were also present in this set up.

The determination of the amounts of the respective cell types in the total cell population per set up was carried out through flow cytometry of three experimental set ups each (see Figure 3). The results are reported as mean values including the standard deviation.

CD14⁺/CD3⁺-cells can neither be determined in the set up "Mo" nor in the set up "Mo+Ly" at the beginning of the culture (d 0 = day 0). After a cultivation period of five days, the experiment was terminated and the cells were characterised by FACS-analysis after detaching the cells from the bottom of the tissue culture flask as described in Example 2. It was found that the relative amount of CD14⁺-cells decreases in these set ups, from 92 % to 42 % in set up "Mo" and from 45 % to 28 % in set up "Mo+Ly". Seemingly, the lymphocytes proliferate quicker than the monocytes; the relative amount of lymphocytes increased in set up "Mo" from 4.8 % to 69.8 % and in set up "Mo+Ly" from 23.5 % to 50.6 %. During the cultivation, CD14⁺/CD3⁺-cells effective as TAIC are formed in both cultures. It is important in this context that a significantly higher increase in CD14⁺/CD3⁺-cells is observed in the set up "Mo+Ly" with an amount of 32.0 ± 5.3 % as opposed to set up "Mo" with only 7.2 ± 3.2 %.

These results demonstrate that the purification of the cells for enrichment of monocytes to a relative amount of more than 90 % at the beginning of the cultivation has a negative influence on the generation of the immuno suppressive CD14⁺/CD3⁺-cells in the TAIC population, while the method described in Examples 1 and 2 leads to a significantly higher yield of CD14⁺/CD3⁺-cells.

### Example 5

### Determination of the Influence of the IDO Inhibitor 1-methyltryptophane on the Immunosuppressing Activity of the TAIC

In order to clarify, whether the inhibitor of the enzyme indolamine-2,3-dioxygenase (IDO) 1-methyltryptophane (1-MT) influences the suppressor function of the TAIC generated in the "Mo" and "Mo+Ly" set ups (see Example 4), a variety of mixed lymphocyte cultures (MLC) with PHA (phytohaemagglutinine) stimulated T-cells in the presence and in the absence of 1-MT were established.

In these mixed lymphocyte cultures 50.000 lymphocytes with 2 µg PHA were transferred into the wells of a 96-well-plate, followed by a proliferation period over 144 hours (indicated as "PhaLy"). Lymphocytes only cultivated in medium without addition of PHA were run as a further control (indicated as "Ly").

In order to determine the proliferation of PHA-stimulated lymphocytes in the different co-cultures, the following 4 set ups were run and examined:
- PhaLy + "Mo+Ly": PHA-stimulated lymphocytes and TAIC [10⁵ cells from the set up "Mo+Ly" according to Example 4].
- PhaLy+"Mo+Ly"+1-MT: PHA-stimulated lymphocytes and TAIC in the presence of 2 µmol 1-MT [10⁵ cells from the set up "Mo+Ly" according to Example 4].
- PhaLy + "Mo": PHA-stimulated lymphocytes and TAIC [10⁵ cells from the set up "Mo" according to Example 4].
- PhaLy+"Mo"+1-MT: PHA-stimulated lymphocytes and TAIC in the presence of 2 µmol 1-MT [10⁵ cells from the set up "Mo" according to Example 4].

After 144 hours cultivation, all controls or co-cultures were further cultivated for 24 hours in the presence of ³[H]-thymidine ("pulsed") and thereafter the amount of radioactively labeled thymidine incorporated was determined as counts per minute (cpm), see Figure 4. In this arrangement, the amount of the radio activity determined is a measure for the amount of labeled thymidine incorporated into the DNA, and therefore a measure for the proliferation rate of the lymphocytes. The values reported in Figure 4 correspond to mean values from triple determinations of 3 experiments each with indication of the standard deviation.

The results demonstrated that lymphocytes not stimulated with PHA ("Ly") do not significantly proliferate, the mean radioactivity observed being 367 cpm (see Figure 4). In contrast, the stimulation with 2 µg PHA leads to a significant increase in the proliferation rate of the lymphocytes ("PhaLy"), the highest incorporation being measured at a mean value of 18459 cpm in these samples.

The addition of TAIC to the lymphocyte cultures clearly decreased the proliferation rate strongly if cells from the set up "Mo+Ly" from Example 4 were added (PhaLy + "Mo+Ly", determined value 1498 cpm), and less strongly when cells from the set up "Mo" from Example 4 were added (PhaLy + "Mo", measured value 3369 cpm).

The results obtained after addition of 2 µmol 1-methyltryptophane (1-MT) to the set ups containing "Mo+Ly" and "Mo" with stimulated lymphocytes demonstrated that 1-methyltryptophane (1-MT) increases the suppressor function of the TAIC synergistically, whereby the decrease was stronger with the TAIC from the set up "Mo+Ly" (measured value 267cpm) than with the TAIC from the set up "Mo" (measured value 390 cpm).

### Example 6

### In vitro co-cultivation of human TAIC with allogeneic lymphocytes for generating regulatory T-cells

To examine whether TAIC induce the formation of regulatory T-cells, i.e. CD4⁺/CD25⁺-lymphocytes, in the recipient, several different *in vitro* cultures of TAIC with lymphocytes were run and the formation of regulatory T-cells resulting therefrom was analyzed.

For this purpose, TAIC of donor A were directly or indirectly co-cultivated *in vitro* together with lymphocytes of a donor B. In the direct co-culture, the direct cell-to-cell-contact between the TAIC (of donor A) and the lymphocytes (of donor B) was made possible, while in the indirect co-culture a membrane ("cell culture insert", 0.4 µm pore size, Falcon, order no. 353090) allowed exchange of the medium, but inhibited the physical contact of the two cell populations. The direct or indirect co-culture is carried out for preferably 3 to 5 days, more preferably for 4 days under incubator conditions i.e. at 37°C and in a 5% CO₂ atmosphere.

After culturing, the respective numbers of regulatory T-cells (CD4⁺/CD25⁺) were determined in both set ups as well as in the controls, wherein TAIC or lymphocytes respectively were cultivated alone. Further, the number of CD3⁺/CD14⁺-cells, which represent the component of the TAIC cell population having the most significant suppressor function in the mixed lymphocyte culture, was determined in the control set up, wherein the TAIC were cultured alone.

In all set ups, the surface antigens of the cells were determined per FACS analysis and the amount of the respective cell populations in the total amount of the cells was analysed.

Further, the relative expression of three new master genes specifically expressed by regulatory T-cells (Foxp3, CTLA-4 and Integrin α_{E}β₇) was determined by PCR in the respective cell population (see Table). Foxp3 is a specific transcription factor, which is viewed as a control gene for the development of regulatory T-cells, and which is specifically expressed by these cells [see Hori, S. et al., "Control of Regulatory T-cell Development by the Transcription Factor Foxp3", Science 299, 1057 - 1061 (2003)].

CTLA-4 is a further factor, which is used as a marker for the determination of the regulatory function of CD4⁺/CD25⁺-T-cells (see Khattri, R. et al., "An essential role for Scurfin in CD4+/CD25+ T regulatory cells", Nature Immunology, online publication, doi:10.1038/ni909 (2003); Shimizu, J. et al. "Stimulation of CD25+/CD4+ regulatory T cells through GITR breaks immunological self-tolerance", Nature Immunology, online publication, doi:10.1038/ni759 (2003); Cobbold, S.P. et al. "Regulatory T cells in the induction and maintenance of peripheral transplantation tolerance", Transpl. Int. 16(2), 66-75 (2003)].

Integrin α_{E}β₇ is an integrin, which binds to epithelial Catherine and which may be used as a marker for the most potent sub-population of regulatory CD25⁺-T-cells [see Lehmann, J. et al. "Expression of the integrin αEβ7 identifies unique subsets of CD25+ as well as CD25- regulatory T cells" PNAS 99(20), 13031-13036 (2002)].

The determination of Foxp3-, CTLA-4- and integrin α_{E}β₇-expession was carried out by means of quantitative PCR using GAPDH and β-actin, two "housekeeping genes", as controls; the values determined were, on the one hand, placed in relationship to each other, the value obtained for the CD14⁺/CD3⁻ cells being set at 1; on the other hand the absolute RNA amounts were indicated in µg per µg total RNA, in order to correlate the measured suppression effected by the TAIC *in vitro* and the formation of the CD4⁺/CD25⁺ double positive cells with the expression rate of the respective genes. Standard methods were used for the quantitative PCR, which are known to the skilled person (see Lottspeich, F., Zorbas, H. "Bioanalytik", Spektrum Akademischer Verlag GmbH, Heidelberg-Berlin, 1998).

The Table shows that within the population of lymphocytes obtained from the direct co-culture, the percentage of double positive cells CD4⁺/CD25⁺ increases manifold to a value of 8.7 % in comparison to the amount of CD4⁺/CD25⁺ lymphocytes obtained from the indirect co-culture or control, which is nearly identical with 2.38 % and 2.65 %, respectively.

The sub-population of CD4⁺/CD25⁺ cells expresses the highest relative amounts of mRNA from all tested mastergenes as compared to the expression in CD4⁺/CD25⁻ cells (see Table). While the expression of Foxp3 is increased by about a factor 10 (37 versus 3.75), CTLA-4 expression reaches an even higher maximum expression (4699 versus 0.376). The increase of expression of the third mastergene integrin α_{E}β₇ in the CD4⁺/CD25⁺ cells during co-culturing is nearly as high as for CTLA-4 since the absolute amount of integrin α_{E}β₇ mRNA is raised to

**Table: Determination of the amounts of specific cell populations in the total amount of cells after direct and indirect co-culture by reference to their surface markers CD3, CD4, CD14, CD25, and determination of the relative expression and the absolute amounts of RNA of three genes (Foxp3, CTLA-4 and integrin α_{E}β₇) in these cell populations.**

| | Control | | | | direct co-culture | | indirect co-culture | |
|---|---|---|---|---|---|---|---|---|
| | TAIC | | lymphocytes | | lymphocytes | | lymphocytes | |
| | CD14⁺/ CD3⁺ | CD14⁺/ CD3⁻ | CD4⁺/ CD25⁺ | CD4⁺/ CD25⁻ | CD4⁺/ CD25⁺ | CD4⁺/ CD25⁻ | CD4⁺/ CD25⁺ | CD4⁺/ CD25⁻ |
| FACS [% positive cells] | 41 | 20 | 2.65 | 30,8 | 8.7 | 49 | 2.38 | 27.6 |
| relative Foxp3-expression [PCR] | 50 | 1 | 15 | 1,5 | 37 | 3.76 | 10 | 1.5 |
| absolute RNA-amount Foxp3 | 1.6×10⁻⁸ | 3.2×10⁻¹⁰ | 4.8×10⁻⁹ | 8×10⁻¹⁰ | 1.2×10⁻⁸ | 1.2×10⁻⁹ | 3.2×10⁻⁹ | 4.8×10⁻¹⁰ |
| relative CTLA4-expression [PCR] | 12500 | 1 | 0.375 | 0.1 | 4699 | 0.376 | | |
| absolute RNA-amount CTLA4 | 6,5×10⁻⁶ | 5.2×10⁻¹⁰ | 1.9×10⁻¹⁰ | 5.2×10⁻¹⁰ | 2.4×10⁻⁸ | 1.9×10⁻¹⁰ | | |
| absolute RNA-amount Integrin α_{E}β₇ | 3.4×10⁻⁹ | not detectable | 1.4×10⁻⁹ | 3.4×10⁻¹² | | | | |

1.4 × 10⁻⁹ in CD44⁺/CD25⁺ cells as compared to 3.4 × 10⁻¹² µg mRNA/µg total RNA in CD4⁺/CD25⁻ cells.

After indirect co-culture, the relative amount of Foxp3-mRNA in the CD4⁺/CD25⁺ sub-population amounts to only 10 and is even lower as the relative amount expressed by the lymphocytes of the control, which expresses a relative amount of Foxp3-mRNA of 15.

The relative amount of CTLA-4-mRNA expressed by the lymphocytes of the control is as low as 0.375 in the CD4⁺/CD25⁺-population and 0.1 in the CD4⁺/CD25⁻population.

The expression of CTLA-4 in the CD14⁺/CD3⁺ sub-population of the TAIC cells was similar to the expression of Foxp3 and was also significantly higher than in all other cell populations.

In this regard, it is noteworthy that the CTLA-4 expression (relative value of 12.500) was manifold stronger in the CD14⁺/CD3⁺-sub-population than the Foxp3-expression, which exhibited a relative value of 50.

No expression of the integrin α_{E}β₇ was detectable in the CD14⁺/CD3⁻ sub-population of the TAIC, while similar to the behavior of the expression of the other two analyzed genes, the expression of the integrin in the CD14⁺/CD3⁺-sub-population of the TAIC, measured as absolute value in µg RNA per µg total RNA, reached the highest value (3.4 × 10⁹ µg / µg total RNA).

The significantly increased expression of the three lymphocyte markers Foxp3, CTLA-4 and integrin α_{E}β₇ on the TAIC derived from monocytes as compared to normal lymphocytes is totally unexpected and has to date never been observed on monocytes or on cells derived from monocytes.

In conclusion, the results of this Example as shown above demonstrate the following: If one starts from the assumption that the level of Foxp3-, CTLA-4-, and integrin α_{E}β₇- expression correlates with the immunoregulatory properties of the respective cells, it was demonstrated here, that the suppressor activity of the CD3⁺/CD14⁺ sub-population of the TAIC is associated with a high expression of these three genes. This is even more surprising when it is considered that these markers have to date only been described for lymphocytes, but never in cells of monocytic origin.

It was further shown that the direct co-culture of TAIC with lymphocytes leads to a significantly higher amount of CD4⁺/CD25⁺ lymphocytes as compared to indirect co-cultivation and the cultivation of only lymphocytes. This supports the assumption that also *in vivo* (i.e. in a patient) regulatory T-cells are formed after administration of TAIC. These results are in conformity with the known immunoregulatory function of CD4⁺/CD25⁺ lymphocytes and with the fact, that the contents of Foxp3-, CTLA-4-, and integrin α_{E}β₇-mRNA in these cells are significantly higher than in indirect co-cultivated lymphocytes or in control lymphocytes.

### Example 7

### In vivo Induction of Transplant Tolerance by Lymphocytes co-cultured with TAIC in vitro

The results and conclusions presented in Example 6 were confirmed *in vivo* in an animal experiment. In this example animals in selected inbred combinations were injected with lymphocytes from the recipient which, over a period of 5 days, were previously directly co-cultivated with TAIC from the donor. Other animals were injected with lymphocytes from the recipient, which were cultivated alone in medium as controls.

Animals, which have received autologous co-cultivated lymphocytes from the recipient prior to an allogeneic heart transplantation developed donor specific tolerance, whereas control animals, which have received naive non-cocultivated control lymphocytes from the recipient acutely rejected the transplanted heart within 10 to 14 days:

This is also shown by flow cytometric determination of GM-7-expression in the blood of patients postoperatively prior (left Figure) and after (right Figure) injection of the lymphocytes co-cultivated with TAIC. The fraction of GM-7 binding cells raises from about 0.5 % prior to injection to about 21 % after injection indicating the development of regulatory T-cells capable of binding GM-7.

These results indicate that the physical cell to cell interaction between donor-TAIC and recipient-lymphocytes induces the generation of regulatory T-cells, which, per se, are capable of modifying the syngeneic immune system of the recipients such, that potentially alloreactive T-cells are suppressed and the organ rejection is thereby prevented.

### Example 8

### Use of the self-tolerance inducing cells (STIC) for the treatment of dextran sulphate sodium (DSS)-induced chronic colitis

Colitis can be chemically induced in mice by oral administration of dextran sulphate sodium via drinking water. Administration over a period of 7 days results in the development of acute colitis, repeated administration comprising at least four cycles DSS administration followed by 10 days normal water results in the development of chronic colitis resembling the human colitis ulcerosa.

This system is well established for immunological or molecular examinations [see e.g. Herfarth, H. et al. "Nuclear factor κB activity and intestinal inflammation in dextran sulphate sodium (DSS)-induced colitis in mice is suppressed by gliotoxin" Clin. Exp. Immunol. 120, 59-65 (2000); Egger, B et al. "Mice lacking transforming growth factor α have an increased susceptibility to dextran sulfate-induced colitis" Gastroenterology 113, 825-832 (1997)] and is used herein to examine the potential of STIC in the treatment of the chronic version of DSS-induced colitis resembling the human autoimmune colitis ulcerosa.

Normal female BALB/c mice (20 g each) were kept with unlimited access to water and standard chow. The mice received four cycles of water containing 5 % (w/v) dextran sulphate sodium (DSS; the solution is hereinafter referred to as DSS water) followed by untreated water according to the following scheme:

| | |
|---|---|
| first cycle: | day 1 to 7 DSS water (7 days); |
| | day 8 to 17 normal water (10 days); |
| second cycle: | day 18 to 24 DSS water (7 days); |
| | day 25 to 34 normal water (10 days); |
| third cycle: | day 35 to 41 DSS water (7 days); |
| | day 42 to 51 normal water (10 days); and |
| fourth cycle: | day 52 to 58 DSS water (7 days). |

After day 58 all mice show symptoms of chronic colitis which lasts for at least 2 months (Fig. 6A and 6 B).

The mice were subsequently randomly assigned to four different groups:

| | |
|---|---|
| group 1 (n=5): | on day +1 (day 59) after completion of the fourth cycle of being nourished with DSS water the mice received 62.5 international units heparin in 0.25 ml PBS intravenously (to prevent thrombosis) followed 30 sec later by administration of 5 × 10⁶ STIC from an genetically identical donor mouse (see Example 2) intravenously in 1 ml PBS. |
| group 2 (n=7): | on day +7 (day 65) after completion of the fourth cycle of being nourished with DSS water the mice received 62.5 international units heparin in 0.25 ml PBS intravenously (to prevent thrombosis) followed 30 sec later by administration of 5 × 10⁶ STIC from an genetically identical donor mouse (see Example 2) intravenously in 1 ml PBS. |
| group 3 (n=12): | first control group undergoing the DSS treatment but did not receive any cell injection. |
| group 4 (n=6): | second control group undergoing the DSS treatment; on day +1 (day 59) after completion of the fourth cycle of being nourished with DSS water the mice received 62.5 international units heparin in 0.25 ml PBS intravenously (to prevent thrombosis) followed 30 sec later by administration of 5 × 10⁶ "control cells" from an genetically identical donor mouse (see Example 1) intravenously in 1 ml PBS ("Control cells" are a mix of bone marrow cells, peripheral blood cells and spleen cells representing the cells before culturing to produce the STIC according to example 2, i.e. non-cultivated cells obtained from the spleen of syngeneic mice). |

All mice are sacrificed on day 79, three weeks after completion of the fourth cycle of nourishment with DSS water.

In the course of the experiments the weight of the animals was measured thrice a week during the DSS induction and during the following three weeks. Changes in the body weight of the animals are expressed in percent (weight loss or gain) with reference to the weight of the mice at day 59, when they received the cell therapy. Changes in the range of about 5 to 15 % are considered to be significant.

The' changes in the body weight of the mice in the course of the 3 week period after the end of the DSS treatment are shown in Fig. 7. As can bee seen, the administration of STIC on day +1 (group 1 = □) leads to an adequate age-dependent gain of weight, that can neither be found in untreated animals (group 3 = ▼), nor in animals treated with "control cells" at day +1 (group 4 = ●) nor in animals treated with STIC at day +7 (group 2 = ▲) during the progression of the disease. The values are mean values obtained from 5 to 7 mice per group, the standard deviation is always below ± 15 %.

In a further experiment, the colon tissue was histologically stained with Hematoxylin &Eosin (H&E). The H&E staining was carried out as described by Woods, A.E. and Ellis, R.C. (eds.) (Laboratory Histopathology: A Complete Reference, vol. 1 & 2; Churchill & Livingstone, 1994). The condition of the stained mucosa was scored by two independently acting pathologists that were blinded with respect to the experimental design according to the following scheme:

| | |
|---|---|
| score 0 | healthy unobtrusive finding; |
| score 1 | minimal colitis; |
| score 2 | moderate colitis; |
| score 3 | heavy colitis; and |
| score 4 | ulcerative colitis accompanied by destruction of the whole mucosa. |

The results of the scoring are shown in Figure 8. Three weeks after completion of the fourth circle of feeding with DSS water the control animals of group 3 (no cell injection) have an average score of about 4, whereas the animals of group 1 (administration of STIC on day +1) or group 2 (administration of STIC on day +7) have significantly reduced average scores of about 1 (group 1) and about 1.5 (group 2), respectively. The animals treated with "control cells" (group 4) have an average score of about 3, which is nearly as high as the score of untreated mice (group 3).

These results are consistent with the findings of a histological evaluation of the stained colon sections (H&E staining, see above) as can be seen in Figure 6. This evaluation is most critical for the determination of the effect of the treatment with STIC. Fig. 6A (magnification × 2.5) and 6B (magnification × 10) illustrate the condition of the colon of an untreated animal of group 3 with a largely destroyed mucosa and the surrounding tissues being heavily invaded by inflammatory cells. These Figures show an advanced stadium of colitis. Contrary to the advanced progression of the colitis in untreated animals (Fig. 6A/B), the morphology of the mucosa is maintained to a large extent in animals of group 1 that received STIC at day +1 (Fig. 6C (magnification × 2.5) and 6D (magnification × 10)). Only minor signs of inflammation and destruction of the mucosa can be seen in these animals, but no invasion of the surrounding tissues. The colon sections of a group 4 animal treated with "control cells" at day +1 (Fig. 6E; magnification × 2.5) and of a group 2 animal treated with STIC at day +7 (Fig. 6F; magnification × 2.5) both show symptoms of advanced colitis with a largely destroyed mucosa and the surrounding tissues being heavily invaded by inflammatory cells similar to those seen in untreated animals (Fig. 6A/B).

The results of the above experiments show that STIC administered at day +1 following the end of the DSS treatment for the induction of colitis are capable of suppressing or clearly alleviating symptoms of colitis. STIC-treated animals do not significantly lose weight (Fig. 7), their histological score is significantly reduced compared to untreated animals (Fig. 8) and the overall condition of the mucosa of the colon is almost normal as can be seen by histological staining of colon sections (Fig. 6 C/D). STIC administered at day +7 (group 2) are not capable of treating the colitis developed during the 7 day period prior to the administration of the STIC. Although there is no reversion of the course of colitis taking place, the progress of the disease is stopped as can be seen from the lower histological score of a group 2 animal compared to an untreated group 3 animal (Fig. 8). This phenomena is specific for the model of DSS-induced colitis used in this example and can not readily be transferred to other conditions.

The mix of bone marrow cells, peripheral blood cells and spleen cells representing the non-cultivated cells obtained from the spleen of syngeneic mice are not feasible for the treatment of colitis, since animals treated with these cells (group 4) show symptoms as severe as untreated animals (Fig. 6 E/F; Fig. 7 and 8). This provides clear evidence that only the STIC are capable of treating colitis and not other cells present in the mix of cells the STIC develop from during culture.

### Example 9

### Use of the self-tolerance inducing cells (STIC) for the treatment of colitis induced in SCID mice by Transfer of CD4⁺CD62L⁺ lymphocytes

The artificial induction of colitis in SCID (severe combined immunodeficiency) mice is another approach for the analysis of the STIC and their potential in the treatment of autoimmune diseases.

SCID mice possess neither T nor B cells. Their immune system can be reconstituted by transfer of a specific subpopulation of thyroid cells. These cells, called CD4⁺CD62L⁺ or CD62L^{high} cells repopulate the immune system but due to lack of control they also induce the development of spontaneous colitis with chronic inflammation. This model system is the so-called "CD62L⁺/CD4⁺ SCID transfer model of chronic experimental colitis in mice" [see e.g. Mudter, J. et al. "A new model of chronic colitis in SCID mice induced by adoptive transfer of CD62L+ CD4+ T cells: Insights into the regulatory role of interleukin-6 on apoptosis" Pathobiology 70, 170-176 (2002)] that allows more specific analysis of cell populations involved in the development in colitis as an example of autoimmune diseases.

The model is used herein to examine the potential of STIC in the treatment of colitis induced by an deregulated immune system. This model allows more mechanistic studies, but is considered to be the weaker system compared to the DSS model system (see Example 8 above) because the immune system is not fully repopulated.

SCID BALB/c mice (18 to 22 g each) receive an intraperitoneal injection of 1 × 10⁶ CD4⁺/CD62L-selectin^{high} T cells isolated from spleens of normal BALB/c mice using the "Macs" magnetic-bead cell-sorting system (Milteny Biotech, Germany) in PBS. The mice were kept with unlimited access to water and standard chow.

Within 4 to 6 weeks after transfer of the CD4⁺/CD62L-selectin^{high} T cells the mice develop colitis. The development of the colitis is determined by judgement of weight changes.

The mice were subsequently randomly assigned to three different groups:

| | |
|---|---|
| group 1 (n=10): | 6 weeks after initiation of the colitis induction by administration of the CD4⁺/CD62L-selectin^{high} T cells the mice received 62.5 international units heparin in 0.25 ml PBS intravenously (to prevent thrombosis) followed 30 sec later by administration of 5 × 10⁶ STIC from an genetically identical donor mouse (see Example 21) intravenously in 1 ml PBS. |
| group 2 (n=10): | first control group receiving only the CD4⁺/ CD62L-selectin^{hign} T cells but did not receive any further cell injection after 6 weeks. |
| group 3 (n=10): | second control group receiving the CD4⁺/CD62L-selectin^{high} T cells. After 6 weeks the mice received 62.5 international units heparin in 0.25 ml PBS intravenously (to prevent thrombosis) followed 30 sec later by administration of 5 × 10⁶ "control cells" from an genetically identical donor mouse (see Example 2) intravenously in 1 ml PBS. "Control cells" are the same as defined in example 8 (non-cultured mix of bone marrow cells, peripheral blood cells and spleen cells). |

All rats are sacrificed 10 to 12 weeks after transfer of the disease inducing T cells or when the control animals develop signs of colitis judged by weight changes within the basic control group 2.

Similar to the DSS model described in example 8 the body weight of the animals was measured thrice a week during the entire experimental period. Changes in the animal body weight are expressed in percent (weight loss or gain) with reference to the weight of the mice 6 weeks after the beginning of the experiment when they receive the cell therapy. Again, changes in the range of about 5 - 15 % are considered to be significant.

The changes in the body weight of the mice in the course of the entire experimental period are shown in Fig. 9. After 6 weeks mice of all groups are of almost the same body weight. While the administration of STIC after 6 weeks (group 1 = ◆) afterwards leads to an adequate age-dependent gain of weight, mice from the group of untreated animals (group 2 = ■) as well as mice treated with "control cells" after 6 weeks (group 3 = ▲) show a comparatively reduced gain of body weight (group 2) or no gain at all (group 3) during the progression of the disease. The values are mean values obtained from 6 mice per group, the standard deviation is always below ± 15 %.

Immediately after sacrifying the animals the colon length and the spleen weight of these animals were determined. Both values allow an estimation of the extent of inflammation, but are not very sensitive.

Figure 10 illustrates the results of the measurements of the colon length (Fig. 10A) and the spleen weight (Fig. 10B). As can be seen in this Figure, the colons of animals treated with STIC (group 1) are significantly longer (about 11 cm) and their spleen significantly smaller (about 50 mg) than that of control animals. The control animals of group 2 have shortened colons (about 10.3 cm) and enlarged spleens (about 100 mg) indicative for severe inflammation, whereas the animals receiving control cells (group 3) develop less severe symptoms of colitis (colon lengths of about 10.6 cm and spleen weights of about 60 mg) compared to the control animals (group 2), but a more advanced colitis compared to the animals treated with the STIC (group 1).

Subsequently, in a further experiment, the colon tissue was histologically stained with Hematoxylin & Eosin (H&E staining; Woods, A.E. and Ellis, R.C. (eds.); Laboratory Histopathology: A Complete Reference, vol. 1 & 2; Churchill & Livingstone, 1994). As already described in Example 8, the condition of the mucosa was scored by two independently acting pathologists, that were blinded with respect to the experimental design. The scoring scheme for the mucosa was the same as in Example 8:

| | |
|---|---|
| score 0 | healthy unobtrusive finding; |
| score 1 | minimal colitis; |
| score 2 | moderate colitis; |
| score 3 | heavy colitis; and |
| score 4 | ulcerative colitis accompanied by destruction of the whole mucosa. |

The average score is shown in Figure 11. Six weeks after transfer of the treatment the control animals of group 2 receiving no cell injection have an average score of about 3 indicating heavy colitis. The mice of group 1 receiving STIC and the mice of group 2 receiving control cells after 6 weeks have significantly reduced average scores of about 1.5 (indicating minimal colitis; group 1) and about 2.5 (indicating moderate colitis; group 2), respectively.

These results are again consistent with the findings of a histological evaluation of the colon sections stained with H&E as can be seen in Figure 12 (magnification x 100). Fig. 12 A/B illustrate the condition of colons of animals of group 3 which remained untreated after induction of colitis with CD62L^{high} T cells. Both examples show marked infiltration with mononuclear cells and substantial loss of mucosal integrity, crypt damage and infiltration of the submucosa and muscularis propria layer. Injection of "control cells" (non-M-CSF and γ-interferon-stimulated monocytes derived from blood, bone-marrow and or spleen tissue) as described did not prevent mononuclear cell infiltration and mucosal loss as can be seen in Fig. 12C/D. Both specimen also show substantial submucosal infiltration with lymphocytes, but the muscularis propria is still intact. Administration of STIC after colitis induction with CD62L^{high} T cells profoundly ameliorated the mucosal integrity (Fig. 12E/F). In colon sections of both specimen only minor lymphocyte infiltration, no crypt loss and no damage to the submucosa and muscularis propria layers within the analysed bowel segments can be seen.

Similar to the results obtained for the administration of STIC for the treatment of dextran sulphate sodium (DSS)-induced chronic colitis (see Example 8 above) it is shown here using the CD62L⁺/CD4⁺ SCID transfer model of chronic experimental colitis in mice that STIC are capable of suppressing or clearly alleviating symptoms of the induced colitis. STIC-treated mice show an adequate age-dependent gain of weight (Fig. 9), their colon is significantly longer (Fig. 10A) and their spleen weight significantly lower (Fig. 10B) than that of mice of the control group suffering from severe inflammation of the colon. Finally, the histological score of STIC-treated mice is significantly lower compared to untreated animals (Fig. 11) and the histological analysis of colon sections of mice treated with STIC shows only minor to no symptoms of colitis (Fig. 12).

Compared with the effect of the treatment of colitis with STIC the mix of bone marrow cells, peripheral blood cells and spleen cells representing the non-cultivated cells obtained from the spleen of syngeneic mice exhibits a significantly reduced efficacy, since animals treated with these cells (group 3) show more severe symptoms than that found in animals treated with STIC (Fig. 9, 10A/B, 11 and 12). Contrary to the results obtained in Example 8 using the DSS model system, in the model of colitis induction with CD62L^{high} cells the control cells are able to alleviate the symptoms to some extent, since the animals receiving the control cells exhibit less severe symptoms compared to the control animals that do not receive any cells.

In accordance with the results of Example 8, this again provides clear evidence that the STIC of the invention are capable of treating autoimmune diseases.

## Claims

1. A process for the preparation of cells for the prevention and/or treatment of diseases associated with disturbed self-tolerance in a patient, **characterised in that**
a) monocytes isolated from the blood of the patient to whom the cells are to be administered are in vitro multiplied in a suitable culture medium which contains the cellular growth factor M-CSF;
b) the monocytes are cultivated simultaneously with or following step a) in a culture medium containing γ-IFN; and
c) the cells formed in step b) are obtained by separating the cells from the culture medium.

2. A process according to claim 1 **characterised in that** the monocytes are of human origin.

3. A process according to claims 1 or 2 **characterised in that** the monocytes are isolated from the blood in such a manner that next to the monocytes also lymphocytes are present in an amount of at least 10% by reference to the total cell number in the isolate.

4. A process according to claims 1 to 3, **characterised in that** the cells formed in step b) or obtained in step c) are selected by binding to the antibody produced by the hybridoma cell line DSM ACC2542.

5. A process according to claims 1 to 4, **characterised in that** among the cells formed in step b) or obtained in step c) of claim 1 or obtained in the selection step according to claim 4, those cells are selected which co-express the antigens CD3 and CD14 on their cell surface.

6. A process according to claims 1 to 5, **characterised in that** the M-CSF concentration in the culture medium is 1 to 20 µg/l.

7. A process according to claims 1 to 6, **characterised in that,** subsequent to step a) the monocytes are cultivated for 24 to 72 hours in a culture medium containing γ-IFN, the cultivation in the presence of γ-IFN beginning 3 to 6 days after the beginning of cultivation step a).

8. A process according to claim 7, **characterised in that** the γ-IFN concentration in the culture medium is 0.1 to 20 ng/ml.

9. A process according to claims 1 to 8 **characterised in that** the total cultivation period in steps a) and b) is 4 to 8 days.

10. A process according to claims 1 to 9, **characterised in that** subsequent to step c) of claim 1, or subsequent to the selection steps according to claims 4 and 5, the cells are suspended in a suitable cell culture medium or in a PBS or NaCl solution.

11. A process according to claims 1 to 10, **characterised in that** the cells are suspended in a freezing medium and are subsequently deep frozen.

12. A process according to claim 11, **characterised in that** the freezing medium comprises fetal calf serum (FCS) or human ABO compatible serum and DMSO.

13. A process according to claims 1 to 10, **characterised in that** the cells obtained from step c) of claims 1 to 3 or 6 to 10, or the cells obtained from the selection steps of claims 4 or 5 are formulated into a pharmaceutical preparation.

14. Cells co-expressing the antigens CD3 and CD14 on their cell surface for the prevention and/or treatment of diseases associated with disturbed self-tolerance in patient, obtainable by any of the processes according to claims 1 to 12.

15. Cells according to claim 14, **characterised in that** they are of human origin.

16. Cell preparation containing the cells according to claims 14 or 15 in a suitable medium.

17. Pharmaceutical composition containing cells of monocytic origin, co-expressing the antigens CD3 and CD14 on their cell surface, for the prevention and/or the treatment of diseases associated with disturbed self-tolerance in a patient.

18. Pharmaceutical composition containing the cells according to claims 14 or 15 or the cell preparation according to claim 16, or the cells obtained from step c) of claim 1 or the cells obtained from the selection step of claim 4.

19. Pharmaceutical composition according to claims 17 and 18 for the prevention and/or the treatment of autoimmune diseases.

20. Pharmaceutical composition according to claims 17 and 18 for the prevention and/or the treatment of allergies.

21. Use of the cells according to claims 14 or 15, or of the cells obtained from step c) of claims 1 to 3 or 6 to 10, or of the cells obtained from the selection steps of claims 4 or 5, or of the cell preparation according to claim 16 for manufacturing a pharmaceutical composition for the prevention and/or treatment of diseases associated with disturbed self-tolerance.

22. Use according to claim 21 for the prevention and/or treatment of autoimmune diseases.

23. Use of claim 22, **characterised in that** the autoimmune disease is one or more of the diseases selected from rheumatic diseases with autoimmune features, diabetes mellitus, autoimmune diseases of the blood and blood vessels, autoimmune diseases of the liver, autoimmune diseases of the thyroid, autoimmune diseases of the central nervous system, and bullous skin diseases. ,

24. Use according to claim 21 for the prevention and/or treatment of allergies.

25. Use according to claim 24, **characterised in that** the allergy is selected from allergies induced by non-self proteins, organic substances and/or inorganic substances.

26. Use according to claim 25, **characterised in that** the allergy is selected from hayfever and/or allergies induced by drugs, chemicals, viruses, bacteria, fungi, food components, metals, gases, animal skin scale, hair and/or animal excreta.

27. The use of self-tolerance inducing cells according to claims 14 or 15, or of the cells obtained from step c) of claims 1 to 3 or 6 to 10, or of the cells obtained from the selection steps of claims 4 or 5, or of the cell preparation of claim 16, for *in vitro* generating and/or propagating autologous regulatory T-lymphocytes.

28. The use according to claim 27, wherein the regulatory T-lymphocytes co-express the antigens CD4 and CD25 on their cell surface.

29. A process for the generation and/or propagation of autologous regulatory T-lymphocytes, **characterised in that** self-tolerance inducing cells according to claims 14 or 15, or the cells obtained from step c) of claims 1 to 3 or 6 to 10, or the cells obtained from the selection steps of claims 4 or 5, or the cell preparation of claim 16 are co-cultivated with an autologous T-lymphocyte preparation.

30. A process according to claim 29, wherein the regulatory T-lymphocytes are obtained from the culture medium.

31. A process according to claims 29 or 30, **characterised in that** the regulatory T-lymphocytes co-express the antigens CD4 and CD25 on their cell surface.

32. A process according to claims 29 or 31, **characterised in that** the regulatory T-lymphocytes are obtained from the culture medium by FACS sorting.

33. The use of the antibodies produced by the hybridoma cell line DSM ACC2542 for the detection and/or selection of cells obtained by the process of claims 1 to 14 suitable for the prevention and/or treatment of diseases associated with disturbed self-tolerance in a patient.

## Patentansprüche

1. Verfahren zur Herstellung von Zellen für die Prävention und/oder Behandlung von Krankheiten, die mit gestörter Selbst-Toleranz in einem Patienten einhergehen, **dadurch gekennzeichnet, dass** man
a) Monozyten, die aus dem Blut desjenigen Patienten isoliert wurden, dem die Zellen verabreicht werden sollen, *in vitro* in einem geeigneten Kulturmedium vermehrt, welches den Zellwachstumsfaktor M-CSF enthält;
b) die Monozyten gleichzeitig oder im Anschluss an Stufe a) in einem Kulturmedium kultiviert, das γ-IFN enthält; und
c) die in Stufe b) gebildeten Zellen **dadurch** gewinnt, dass man die Zellen von dem Kulturmedium abtrennt.

2. Verfahren nach Anspruch 1, **dadurch gekennzeichnet, dass** die Monozyten humanen Ursprungs sind.

3. Verfahren nach einem der Ansprüche 1 oder 2, **dadurch gekennzeichnet, dass** die Monozyten auf solche Weise aus dem Blut isoliert werden, dass neben den Monozyten auch Lymphozyten in einer Menge von mindestens 10 %, bezogen auf die Gesamtzellzahl in dem Isolat, vorhanden sind.

4. Verfahren nach den Ansprüchen 1 bis 3, **dadurch gekennzeichnet, dass** man die in Stufe b) gebildeten oder in Stufe c) gewonnenen Zellen durch Bindung an den Antikörper selektiert, der von der Hybridomzelllinie DSM ACC2542 produziert wird.

5. Verfahren nach den Ansprüchen 1 bis 4, **dadurch gekennzeichnet, dass** man aus den in Stufe b) gebildeten oder in Stufe c) von Anspruch 1 gewonnenen Zellen, oder aus den in dem Selektionsschritt gemäß Anspruch 4 erhaltenen Zellen, diejenigen Zellen auswählt, welche die Antigene CD3 und CD14 auf ihrer Zelloberfläche co-exprimieren.

6. Verfahren nach den Ansprüchen 1 bis 5, **dadurch gekennzeichnet, dass** die M-CSF-Konzentration in dem Kulturmedium 1 bis 20 µg/l beträgt.

7. Verfahren nach den Ansprüchen 1 bis 6, **dadurch gekennzeichnet, dass** man im Anschluss an Stufe a) die Monozyten 24 bis 72 Stunden lang in einem Kulturmedium kultiviert, welches γ-IFN enthält, wobei die Kultivierung in Gegenwart von γ-IFN 3 bis 6 Tage nach Beginn der Kultivierungsstufe a) beginnt.

8. Verfahren nach Anspruch 7, **dadurch gekennzeichnet, dass** die γ-IFN-Konzentration in dem Kulturmedium 0,1 bis 20 ng/ml beträgt.

9. Verfahren nach den Ansprüchen 1 bis 8, **dadurch gekennzeichnet, dass** die gesamte Kultivierungsdauer in den Stufen a) und b) 4 bis 8 Tage beträgt.

10. Verfahren nach den Ansprüchen 1 bis 9, **dadurch gekennzeichnet, dass** man im Anschluss an Stufe c) von Anspruch 1, oder anschließend an die Selektionsstufen gemäß den Ansprüchen 4 und 5, die Zellen in einem geeigneten Zellkulturmedium oder in einer PBS- oder NaCl-Lösung suspendiert.

11. Verfahren nach den Ansprüchen 1 bis 10, **dadurch gekennzeichnet, dass** man die Zellen in einem Gefriermedium suspendiert und anschließend tiefgefriert.

12. Verfahren nach Anspruch 11, **dadurch gekennzeichnet, dass** das Gefriermedium fötales Kälberserum (FCS) oder human ABO kompatibles Serum und DMSO enthält.

13. Verfahren nach den Ansprüchen 1 bis 10, **dadurch gekennzeichnet, dass** die aus Stufe c) der Ansprüche 1 bis 3 oder 6 bis 10 erhaltenen Zellen, oder die aus den Selektionsstufen der Ansprüche 4 und 5 erhaltenen Zellen als pharmazeutisches Präparat formuliert werden.

14. Zellen, welche die Antigene CD3 und CD14 auf ihrer Zelloberfläche co-exprimieren, für die Prävention und/oder Behandlung von Erkrankungen, die mit gestörter Selbst-Toleranz in einem Patienten einhergehen, herstellbar nach jeglichem der Verfahren gemäß den Ansprüchen 1 bis 12.

15. Zellen gemäß Anspruch 14, **dadurch gekennzeichnet, dass** sie humanen Ursprungs sind.

16. Zellpräparat, welches die Zellen gemäß den Ansprüchen 14 oder 15 in einem geeigneten Medium enthält.

17. Pharmazeutische Zusammensetzung, welche Zellen monozytären Ursprungs enthält, die auf ihrer Zelloberfläche die Antigene CD3 und CD14 exprimieren, zur Prävention und/oder Behandlung von Erkrankungen, die mit gestörter Selbst-Toleranz in einem Patienten einhergehen.

18. Pharmazeutische Zusammensetzung, welche die Zellen nach den Ansprüchen 14 oder 15 oder das Zellpräparat nach Anspruch 16, oder die Zellen, die aus der Stufe c) von Anspruch 1 gewonnen wurden, oder die Zellen enthält, die aus der Selektionsstufe gemäß Anspruch 4 erhalten wurden.

19. Pharmazeutische Zusammensetzung gemäß den Ansprüchen 17 und 18 zur Prävention und/oder Behandlung von Autoimmunerkrankungen.

20. Pharmazeutische Zusammensetzung nach den Ansprüchen 17 und 18 zur Prävention und/oder Behandlung von Allergien.

21. Verwendung der Zellen nach den Ansprüchen 14 oder 15, oder der Zellen, die aus der Stufe c) der Ansprüche 1 bis 3 oder 6 bis 10 erhalten wurden, oder der Zellen, die aus der Selektionsstufe der Ansprüche 4 oder 5 erhalten wurden, oder des Zellpräparats gemäß Anspruch 16, zur Herstellung einer pharmazeutischen Zusammensetzung für die Prävention und/oder Behandlung von Erkrankungen, die mit gestörter Selbst-Toleranz assoziiert sind.

22. Verwendung nach Anspruch 21 zur Prävention und/oder Behandlung von Autoimmunerkrankungen.

23. Verwendung nach Anspruch 22, **dadurch gekennzeichnet, dass** es sich bei der Autoimmunerkrankung um eine oder mehrere der Erkrankungen ausgewählt aus rheumatischen Erkrankungen mit Autoimmunmerkmalen, Diabetes mellitus, Autoimmunerkrankungen des Bluts und der Blutgefäße, Autoimmunerkrankungen der Leber, Autoimmunerkrankungen der Schilddrüse, Autoimmunerkrankungen des zentralen Nervensystems und bullöse Hauterkrankungen handelt.

24. Verwendung gemäß Anspruch 21 zur Prävention und/oder Behandlung von Allergien.

25. Verwendung gemäß Anspruch 24, **dadurch gekennzeichnet, dass** die Allergie ausgewählt ist aus Allergien die durch nichtselbst Proteine, organische Substanzen und/oder anorganische Substanzen ausgelöst sind.

26. Verwendung gemäß Anspruch 25, **dadurch gekennzeichnet, dass** die Allergie ausgewählt ist aus Heuschnupfen und/oder Allergien, die durch Medikamente, Chemikalien, Viren, Bakterien, Pilze, Nahrungsmittelkomponenten, Metalle, Gase, Tierhautschuppen, Haar und/oder Tierexkremente ausgelöst sind.

27. Verwendung der Selbst-Toleranz induzierenden Zellen gemäß den Ansprüchen 14 und 15, oder der aus Stufe c) der Ansprüche 1 bis 3 oder 6 bis 10 gewonnenen Zellen, oder der Zellen, die aus den Selektionsstufen der Ansprüche 4 oder 5 erhalten wurden, oder des Zellpräparats gemäß Anspruch 16, zur in vitro-Generation und/oder Propagierung autologer regulatorischer T-Lymphozyten.

28. Verwendung nach Anspruch 27, **dadurch gekennzeichnet, dass** die regulatorischen T-Lymphozyten die Antigene CD4 und CD25 auf ihrer Zelloberfläche co-exprimieren.

29. Verfahren zum Generieren und/oder Propagieren autologer regulatorischer T-Lymphozyten, **dadurch gekennzeichnet, dass** man Selbst-Toleranz induzierende Zellen gemäß den Ansprüchen 14 oder 15, oder die aus der Stufe c) der Ansprüche 1 bis 3 oder 6 bis 10 gewonnenen Zellen, oder die aus der Selektionsstufe der Ansprüche 4 oder 5 erhaltenen Zellen, oder das Zellpräparat gemäß Anspruch 16 mit einer autologen T-Lymphozytenpräparation co-kultiviert.

30. Verfahren nach Anspruch 29, **dadurch gekennzeichnet, dass** die regulatorischen T-Lymphozyten aus dem Kulturmedium gewonnen werden.

31. Verfahren nach den Ansprüchen 29 oder 30, **dadurch gekennzeichnet, dass** die regulatorischen T-Lymphozyten die Antigene CD4 und CD25 auf ihrer Zelloberfläche co-exprimieren.

32. Verfahren nach den Ansprüchen 29 oder 31, **dadurch gekennzeichnet, dass** man die regulatorischen T-Lymphozyten durch FACS-Sortierung aus dem Kulturmedium gewinnt.

33. Verwendung der von der Hybridomzelllinie DSM ACC2542 produzierten Antikörper zum Nachweis und/oder der Selektion von Zellen, die nach dem Verfahren der Ansprüche 1 bis 14 hergestellt wurden, und die zur Prävention und/oder Behandlung von Krankheiten geeignet sind, die mit gestörter Selbst-Toleranz in einem Patienten einhergehen.

## Revendications

1. Procédé de préparation de cellules destinées à la prévention et/ou au traitement de maladies associées à une auto-tolérance perturbée chez un patient, **caractérisé en ce que**
a) des monocytes isolés du sang du patient à qui les cellules doivent être administrées sont multipliés *in vitro* dans un milieu de culture approprié qui contient le facteur de croissance cellulaire M-CSF ;
b) les monocytes sont cultivés simultanément à ou après l'étape a) dans un milieu de culture contenant de l'IFN-γ ; et
c) les cellules formées dans l'étape b) sont obtenues en séparant les cellules du milieu de culture.

2. Procédé selon la revendication 1, **caractérisé en ce que** les monocytes sont d'origine humaine.

3. Procédé selon la revendication 1 ou 2, **caractérisé en ce que** les monocytes sont isolés du sang de telle manière qu'à côté des monocytes, des lymphocytes sont également présents dans une quantité d'au moins 10 % en référence au nombre total de cellules dans l'isolat.

4. Procédé selon les revendications 1 à 3, **caractérisé en ce que** les cellules formées dans l'étape b) ou obtenues dans l'étape c) sont sélectionnées par la liaison à l'anticorps produit par la lignée cellulaire d'hybridome DSM ACC2542.

5. Procédé selon les revendications 1 à 4, **caractérisé en ce que** parmi les cellules formées dans l'étape b) ou obtenues dans l'étape c) de la revendication 1 ou obtenues dans l'étape de sélection selon la revendication 4, ces cellules sont sélectionnées par la coexpression des antigènes CD3 et CD14 sur leur surface cellulaire.

6. Procédé selon les revendications 1 à 5, **caractérisé en ce que** la concentration de M-CSF dans le milieu de culture est de 1 à 20 µg/l.

7. Procédé selon les revendications 1 à 6, **caractérisé en ce que,** suite à l'étape a), les monocytes sont cultivés pendant 24 à 72 heures dans un milieu de culture contenant de l'IFN-γ, la culture en présence d'IFN-γ commençant 3 à 6 jours après le début de l'étape a) de la culture.

8. Procédé selon la revendication 7, **caractérisé en ce que** la concentration d'IFN-γ dans le milieu de culture est de 0,1 à 20 ng/ml.

9. Procédé selon les revendications 1 à 8, **caractérisé en ce que** la période totale de culture dans les étapes a) et b) est de 4 à 8 jours.

10. Procédé selon les revendications 1 à 9, **caractérisé en ce que** suite à l'étape c) de la revendication 1, ou suite aux étapes de sélection selon les revendications 4 et 5, les cellules sont mises en suspension dans un milieu de culture cellulaire approprié ou dans une solution de PBS ou de NaCl.

11. Procédé selon les revendications 1 à 10, **caractérisé en ce que** les cellules sont mises en suspension dans un milieu de congélation et sont ensuite profondément congelées.

12. Procédé selon la revendication 11, **caractérisé en ce que** le milieu de congélation comprend du sérum de veau foetal (FCS) ou du sérum humain ABO compatible et du DMSO.

13. Procédé selon les revendications 1 à 10, **caractérisé en ce que** les cellules obtenues à partir de l'étape c) des revendications 1 à 3 ou 6 à 10, ou les cellules obtenues à partir des étapes de sélection de la revendication 4 ou 5 sont formulées dans une préparation pharmaceutique.

14. Cellules coexprimant les antigènes CD3 et CD14 sur leur surface cellulaire pour la prévention et/ou le traitement de maladies associées à une auto-tolérance perturbée chez un patient, qui peuvent être obtenues par l'un quelconque des procédés selon les revendications 1 à 12.

15. Cellules selon la revendication 14, **caractérisées en ce qu'**elles sont d'origine humaine.

16. Préparation cellulaire contenant les cellules selon la revendication 14 ou 15 dans un milieu approprié.

17. Composition pharmaceutique contenant des cellules d'origine monocytaire, coexprimant les antigènes CD3 et CD14 sur leur surface cellulaire, pour la prévention et/ou le traitement de maladies associées à une auto-tolérance perturbée chez un patient.

18. Composition pharmaceutique contenant les cellules selon la revendication 14 ou 15 ou la préparation cellulaire selon la revendication 16, ou les cellules obtenues à partir de l'étape c) de la revendication 1 ou les cellules obtenues à partir de l'étape de sélection de la revendication 4.

19. Composition pharmaceutique selon les revendications 17 et 18 pour la prévention et/ou le traitement de maladies auto-immunes.

20. Composition pharmaceutique selon les revendications 17 et 18 pour la prévention et/ou le traitement d'allergies.

21. Utilisation des cellules selon la revendication 14 ou 15, ou des cellules obtenues à partir de l'étape c) des revendications 1 à 3 ou 6 à 10, ou des cellules obtenues à partir des étapes de sélection de la revendication 4 ou 5, ou de la préparation cellulaire selon la revendication 16 pour la fabrication d'une composition pharmaceutique destinée à la prévention et/ou au traitement de maladies associées à une auto-tolérance perturbée.

22. Utilisation selon la revendication 21 pour la prévention et/ou le traitement de maladies auto-immunes.

23. Utilisation selon la revendication 22, **caractérisée en ce que** la maladie autoimmune est une ou plusieurs des maladies choisies parmi des maladies rhumatismales avec des caractères auto-immuns, le diabète sucré, des maladies auto-immunes du sang et des vaisseaux sanguins, des maladies auto-immunes du foie, des maladies auto-immunes de la thyroïde, des maladies auto-immunes du système nerveux central et des maladies cutanées bulleuses.

24. Utilisation selon la revendication 21 pour la prévention et/ou le traitement d'allergies.

25. Utilisation selon la revendication 24, **caractérisée en ce que** l'allergie est choisie parmi des allergies induites par des protéines étrangères, des substances organiques et/ou des substances inorganiques.

26. Utilisation selon la revendication 25, **caractérisée en ce que** l'allergie est choisie parmi le rhume des foins et/ou des allergies induites par des médicaments, des substances chimiques, des virus, des bactéries, des champignons, des composants alimentaires, des métaux, des gaz, des desquamations cutanées, des poils d'animaux et/ou des excreta d'animaux.

27. Utilisation des cellules induisant une auto-tolérance selon la revendication 14 ou 15, ou des cellules obtenues à partir de l'étape c) des revendications 1 à 3 ou 6 à 10, ou des cellules obtenues à partir des étapes de sélection de la revendication 4 ou 5, ou de la préparation cellulaire de la revendication 16, pour une génération et/ou une propagation *in vitro* de lymphocytes T régulateurs autologues.

28. Utilisation selon la revendication 27, où les lymphocytes T régulateurs coexpriment les antigènes CD4 et CD25 sur leur surface cellulaire.

29. Procédé de génération et/ou de propagation de lymphocytes T régulateurs autologues, **caractérisé en ce que** les cellules induisant une auto-tolérance selon la revendication 14 ou 15, ou les cellules obtenues à partir de l'étape c) des revendications 1 à 3 ou 6 à 10, ou les cellules obtenues à partir des étapes de sélection de la revendication 4 ou 5, ou la préparation cellulaire de la revendication 16 sont co-cultivées avec une préparation de lymphocytes T autologues.

30. Procédé selon la revendication 29, dans lequel les lymphocytes T régulateurs sont obtenus à partir du milieu de culture.

31. Procédé selon la revendication 29 ou 30, **caractérisé en ce que** les lymphocytes T régulateurs coexpriment les antigènes CD4 et CD25 sur leur surface cellulaire.

32. Procédé selon la revendication 29 ou 31, **caractérisé en ce que** les lymphocytes T régulateurs sont obtenus à partir du milieu de culture par la technique de tri FACS.

33. Utilisation des anticorps produits par la lignée cellulaire d'hybridome DSM ACC2542 pour la détection et/ou la sélection des cellules obtenues par le procédé des revendications 1 à 14, approprié pour la prévention et/ou le traitement de maladies associées à une auto-tolérance perturbée chez un patient.
